# EUROPEAN PATENT SPECIFICATION

(11) **EP 1 165 132 B1**
(45) Date of publication and mention of the grant of the patent: **19.10.2011**
(21) Application number: 00921585.6
(22) Date of filing: 10.04.2000
(51) Int. Cl.: A61K 39/39, A61K 48/00, A61K 9/00, A61P 35/00, A61P 31/00, A61P 33/00

(54) **DRY FORMULATION FOR TRANSCUTANEOUS IMMUNIZATION**
TROCKENFORMULIERUNG FÜR TRANSKUTANE IMMUNISIERUNG
FORMULATION SECHE POUR IMMUNISATION TRANSCUTANEE

(30) Priority: 08.04.1999 US 128370 P; 07.04.2000 US 545417
(43) Date of publication of application: 02.01.2002
(60) Divisional application: 10180720.4
(73) Proprietor: Intercell USA, Inc., Gaithersburg, MD 20878 (US)
(72) Inventor: Glenn, Gregory M., Cabin John, MD 20818 (US); Scharton-Kersten, Tanya, Cary, NC 27513 (US)
(74) Representative: Vossius & Partner
(86) International application number: PCT/US2000/008714
(87) International publication number: WO 2000/061184

(56) References cited:
- EP-A- 0 891 770
- WO-A-98/20734
- WO-A-98/42375
- WO-A-99/43350
- GLENN G M ET AL: "Transcutaneous immunization with bacterial ADP-ribosylating exotoxins as antigens and adjuvants" INFECTION AND IMMUNITY,US,AMERICAN SOCIETY FOR MICROBIOLOGY. WASHINGTON, vol. 67, no. 3, March 1999 (1999-03), pages 1100-1106, XP002110054 ISSN: 0019-9567 cited in the application
- VASSELL ET AL: "Activation of Langerhans cells following transcutaneous immunization" FASEB JOURNAL,US,FED. OF AMERICAN SOC. FOR EXPERIMENTAL BIOLOGY, BETHESDA, MD, vol. 13, no. 4, 12 March 1999 (1999-03-12), page A633 XP002110055 ISSN: 0892-6638
- GLENN ET AL: "Advances in vaccine delivery: transcutaneous immunisation" EXPERT OPINION ON INVESTIGATIONAL DRUGS,GB,ASHLEY PUBLICATIONS LTD., LONDON, vol. 8, no. 6, June 1999 (1999-06), pages 797-805, XP002110056 ISSN: 1354-3784
- KENNEY RICHARD T. ET AL: 'Induction of protective immunity against lethal anthrax challenge with a patch.', 15 August 2004, THE JOURNAL OF INFECTIOUS DISEASES 15 AUG 2004, VOL. 190, NR. 4, PAGE(S) 774 - 782, ISSN 0022-1899
- FRERICHS DAVID M. ET AL: 'Controlled, single-step, stratum corneum disruption as a pretreatment for immunization via a patch.', 23 May 2008, VACCINE 23 MAY 2008, VOL. 26, NR. 22, PAGE(S) 2782 - 2787, ISSN 0264-410X
- FROLOV VLADIMIR G. ET AL: 'Transcutaneous delivery and thermostability of a dry trivalent inactivated influenza vaccine patch' INFLUENZA AND OTHER RESPIRATORY VIRUSES vol. 2, no. 2, March 2008, pages 53 - 60 ISSN: 1750-2640(print)
- KENNEY RICHARD T. ET AL: 'Induction of protective immunity against lethal anthrax challenge with a patch.', 15 August 2004, THE JOURNAL OF INFECTIOUS DISEASES 15 AUG 2004, VOL. 190, NR. 4, PAGE(S) 774 - 782, ISSN 0022-1899
- FRERICHS DAVID M. ET AL: 'Controlled, single-step, stratum corneum disruption as a pretreatment for immunization via a patch.', 23 May 2008, VACCINE 23 MAY 2008, VOL. 26, NR. 22, PAGE(S) 2782 - 2787, ISSN 0264-410X
- FROLOV ET AL: 'Transcutaneous delivery and thermostability of a dry trivalent inactivated influenza vaccine patch' INFLUENZA AND OTHER RESPIRATORY VIRUSES 2008, pages 1 - 8

## Description

### BACKGROUND OF INVENTION

### 1. Field of the Invention

This invention relates to a dry formulation useful for transcutaneous immunization to induce an antigen-specific immune response. In particular, physical forms of the dry formulation include manufactured articles like patches and other solid substrates (e.g., a dressing) used to apply the dry formulation to skin of the subject in need thereof. This formulation is stabilized for storage and transport and, surprisingly, the induced immune response is more robust than with previous liquid formulations.

### 2. Description of the Related Art

Skin, the largest human organ, is an important part of the body's defense against invasion by infectious agents and contact with noxious substances (see Bos, 1997). The skin, however, may also be a target of chronic infections where organisms establish their presence through avoidance of the immune system.

The skin is composed of three layers: the epidermis, the dermis, and subcutaneous fat. The epidermis is composed of the basal, the spinous, the granular, and the cornified layers; the stratum corneum comprises the cornified layer and lipid (Moschella and Hurley, 1992). The principal antigen presenting cells of the skin, Langerhans cells, are reported to be in the mid to upper spinous layers of the epidermis in humans. The dermis contains primarily connective tissue. Blood vessels and lymphatics are confined to the dermis and subcutaneous fat.

The stratum corneum, a layer of dead skin cells and lipids, has traditionally been viewed as a barrier to the hostile world, excluding organisms and noxious substances from the viable cells below the stratum corneum (Bos, 1997). The secondary protection provided by skin antigen presenting cells such as Langerhans cells has only recently been recognized (Celluzzi and Falo, 1997). Moreover, the ability to immunize through the skin using the crucial concept of a skin-active adjuvant has only been recently described (Glenn et al., 1998). Scientific recognition of this important advance in vaccination was prompt. "It's a very surprising result, and it's lovely," said vaccine expert Barry Bloom of the Howard Hughes Medical Institute and the Albert Einstein College of Medicine in New York, the strategy sounds "very easy, very safe, and certainly inexpensive" (CNN News, February 26, 1998).

Another surprising result was the ability to use a toxin as an effective skin-active adjuvant for transcutaneous immunization without adverse effects to the immunized host.

For example, *Vibrio cholerae* secretes cholera toxin (CT) and entertoxogenic *E. coli* (ETEC) secretes heat-labile enterotoxin (LT). These proteins cause intestinal fluid secretion and massive diarrhea (Spangler, 1992) and are viewed as dangerous toxins.

*Vibrio cholera* and cholera toxin (CT) are examples of infectious agents and noxious bacterial products, respectively, which one would have expected the skin to protect against. Craig (1965) reported that stool filtrates of cholera patients injected intracutaneously into rabbits or guinea pigs produced a characteristic delayed onset, sustained edematous induration (swelling), which was induced by the presence of toxin in the skin. The swelling and vascular leakage was so dramatic that it was ascribed to an unknown permeability factor which was later shown to be CT itself. Thus, one could have reasonably expected that CT would be extremely reactogenic when placed on the skin or inserted through the stratum corneum, and would cause similar redness and swelling. The Craig test became a standard measurement for the presence and amount of CT in stool filtrates or culture media. Datta confirmed that this skin reactivity was due to cholera toxin (see Finkelstein and LoSpallutto, 1969).

Craig (1965) cautioned, "The absence of skin lesions in clinical cholera certainly does not preclude the possibility that the noxa responsible for gut damage could also have a deleterious effect upon the skin provided it is applied to skin in sufficient concentration." The extreme reactogenicity of cholera toxin in the skin was used as a test for its toxicity and such prior art evidenced an expectation that cholera toxin would be reactogenic if applied to the skin, producing an undesirable reaction.

In contrast, we have shown cholera toxin to be immunogenic, acting as both antigen and adjuvant, when placed on the skin. It can immunize without any resulting local or systemic side effects. This lack of reactogenicity when cholera toxin was placed on the skin for transcutaneous immunization was surprising and contradicted conclusions one would have drawn from the prior art. A liquid formulation of CT placed on the skin acted as a non-toxic, non-reactogenic adjuvant, in contrast to the expectations of Craig, while injection of CT into the skin results in swelling and redness. Thus, it was not obvious prior to our invention that cholera toxin or other ADP-ribosylating exotoxins would be useful for transcutaneous immunization. See WO 98/20734 and U.S. Pat. Nos. 5,910,306 and 5,980,898.

This expection that cholera toxin or other adjuvants would be highly reactogenic when placed on the skin was further supported by findings using the prototypical adjuvant, Freund's adjuvant. Kleinau et al. (1994) found that topical administration of incomplete Freund's adjuvant on the skin of rats induced arthritis as evidenced clinically and by proliferation of the joint lining, inflammatory infiltrates, and bone and cartilage destruction. They further stated, "This investigation has focused on the arthritogenic role of mineral oil, a prototype for an immunological adjuvant. It is plausible, however, that a number of other compounds with adjuvant properties may also have the same effect when applied percutaneously (sic)." In contrast to this suggestion, we have used a water-in-oil emulsion of a skin-active adjuvant (LT) and found that it safely induced an immune response without any systemic effects. See WO 98/20734 and U.S. Pat. Nos. 5,910,306 and 5,980,898. Thus, it would have been expected that transcutaneous application of adjuvant, and especially an adjuvant in an emulsion, would have produced arthritis from this animal model. Our findings, however, unexpectedly showed that such formulations are devoid of reactogenicity.

Transcutaneous immunization requires both passage of an antigen through the outer barriers of the skin, which was thought to be impervious to such passage, and an immune response to the antigen. Fisher's *Contact Dermatitis* states that molecules of greater than 500 daltons cannot normally pass through the skin. Moreover, according to Hurley, "Skin owes its durability to the dermis, but its chemical impermeability resides in the epidermis and almost exclusively in its dead outer layer, the stratum corneum."

Skin reactions such as allergic or atopic dermatitis are known, but induction of a systemic immune response which elicits antigen-specific immune effectors and provides a therapeutic advantage by simple application of immunogen to skin does not appear to have been taught or suggested prior to our invention.

Generally skin antigen presenting cells (APCs), and particularly Langerhans cells, are targets of sensitization agents which result in pathologies that include contact dermatitis, atopic dermatitis, eczema, and psoriasis. Contact dermatitis may be directed by Langerhans cells which phagocytize antigen, migrate to the lymph nodes, present antigen, and sensitize T cells for the intense destructive cellular response that occurs at the affected skin site (Kripke et al., 1987). An example of atopic dermatitis is a chronic relapsing inflammatory skin disease associated with colonization of the skin with *S. aureus* and thought to be caused by S. aureus-derived superantigens that trigger chronic T-cell mediated skin inflammation through Langerhans cells (Herz et al., 1998; Leung, 1995; Saloga et al.,1996a). Atopic dermatitis may utilize the Langerhans cells in a similar fashion to contact dermatitis, and is generally associated with high levels of IgE antibody (Wang et al., 1996).

In contrast, transcutaneous immunization with cholera toxin or related ADP-ribosylating exotoxins resulted in a novel immune response with an absence of post-immunization skin findings, high levels of antigen-specific IgG antibody, the presence of all IgG subclass antibodies. See WO 98/20734; U.S. Pat. Nos. 5,910,306 and 5,980,898; and U.S. Appln. No. 09/257,188.

There is a report by Paul et al. (1995) of induction of complement-mediated lysis of antigen-sensitized liposomes using transferosomes. The transferosomes were used as a vehicle for antigen, and complement-mediated lysis of antigen-sensitized liposomes was assayed. The limit to passage through the skin by antigen was stated to be 750 daltons. Furthermore, Paul and Cvec (1995) stated that it is "impossible to immunize epicutaneously with simple peptide or protein solutions." Thus, transcutaneous immunization as described herein would not be expected to occur according to this group.

Besides the physical restriction of limiting passage through the skin of low molecular weight, passage of polypeptides was believed to be limited by chemical restrictions. Carson et al. (U.S. Pat. No. 5,679,647) stated that "it is believed that the bioavailability of peptides following transdermal or mucosal transmission is limited by the relatively high concentration of proteases in these tissues. Yet unfortunately, reliable means of delivering peptides ... by transdermal or mucosal transmission of genes encoding for them has been unavailable."

In contrast to transcutaneous immunization, transdermal drug therapy has been understood to target the vasculature found in the dermis. For example, Moschella (1996) states, "The advantages of transdermal therapy over conventional oral administration include: 1. Avoidance of 'peak and trough' plasma concentration profiles. 2. Avoidance of first-pass metabolism in the gastrointestinal tract and liver" (emphasis added). Thus, in the realm of drug delivery, the meaning of transdermal is to pass through the epidermis and into the dermis or lower layers to achieve adsorbtion into the vasculature.

In many cases, effective immunization that leads to protection requires help in the form of adjuvants for the coadministered antigen or plasmid and, therefore, useful immune responses require the use of an adjuvant to enhance the immune response (Stoute et al., 1997; Sasaki et al., 1998). In WO 98/20734 and a subsequent paper (Scharton-Kersten et al., Infect. Immun., in press), we showed that a skin-active adjuvant was required to induce high levels of systemic and mucosal antibodies to coadministered antigens. For example, mice immunized with CT + DT induced high levels of systemic and mucosal anti-DT antibodies. Antibodies are known to correlate with protection against diphtheria. Thus, the skin adjuvant for transcutaneous immunization can be expected to provide 'help' in the immune response to coadministered antigen and to play a critical role in inducing a useful immune response.

Such references explain why our successful use of a molecule like cholera toxin (which is 85,000 daltons) as an antigen-adjuvant in immunization was greeted with enthusiasm and surprise by the art because such large molecules were not expected to pass through the skin and, therefore, would not have been expected to induce a strong, specific immune response.

We have shown in WO 98/20734; U.S. Pat. Nos. 5,910,306 and 5,980,898; and U.S. Appln. No. 09/257,188 that using an ADP-ribosylating exotoxin, such as cholera toxin, heat-labile enterotoxin from *E. coli* (LT), *Pseudomonas* exotoxin A (ETA), and pertussis toxin (PT), could elicit a vigorous immune response which was highly reproducible. Moreover, when such an ADP-ribosylating exotoxin was used as an adjuvant and applied to the skin along with a separate antigen (e.g., bovine serum albumin or diphtheria toxoid) in a saline solution, a systemic and mucosal antigen-specific immune response could be elicited.

Based on this success, we now address the problems of maintaining the activity of a vaccine subjected to harsh conditions during storage or transport, and maintaining sterility in a vaccine formulation prepared in the field. A formulation was needed that could be stored and transported without cold storage and that would minimize the danger of contamination by eliminating the need to mix solutions in the field.

Currently, licensed vaccines are delivered in an aqueous solution or suspension, and administered by the intramuscular or oral route during immunization. The drawbacks of mixing vaccine components with water or buffers under conditions of questionable sterility and the possibility that antigens in solution will break down are well known and, in part, has led to the need for cold storage of vaccine components. Vaccine components in the presence of water are chemically less stable and more prone to contamination through the provision of an aqueous medium for the growth of bacteria. The stringent requirement for cold storage during transport and storage of vaccines has led to the 'cold chain', indicating that at all times after manufacture of the vaccine, the vaccine is kept in proper cold storage conditions. This increases the complexity of storing vaccine, creates logistical problems when transporting vaccine, and adds greatly to the expense of vaccination. A study reported by John *et al* (1996) illustrates the problems and expenses of cold chain maintenance:
"Infant immunisation coverages of 80-85% have become sustainable throughout the developing world. The vaccine market is enormous in developing countries; in 1992, the total consumption of vaccine doses amounted to 2207 millions. There were only 127 million births, and the actual doses given to infants would have been fewer than 1000 million. This indicates huge wastage, which could be saved if vaccines were more stable at high temperatures and did not become contaminated".

The present inventors have demonstrated previously (WO 98/20734 and Glenn et al, Infection and Immunity 67, 1100-1106, 1999) that aqueous formulations comprising bacterial ADP-ribosylating exotoxins or their subunits applied to intact skin may act as adjuvants to enhance the immune response to antigens delivered transcutaneously.

Vassell et al (FASEB J, 13, A633, 1999*)* described the activation of Langerhans cells in the skin following transcutaneous immunization.

WO 98/42375 discloses detoxified mutants of various bacterial ADP-ribosylating exotoxins, especially those from pertussis (PT), cholera (CT) and E. coli (LT) and their use as parenteral adjuvants.

EP 0891 770 discloses the preparation of an ethanolic solution of a low molecular weight peptide antigen, which is applied to adhesive tape and allowed to dry. The tape is intended for transcutaneous immunisation. The formulation does not comprise any adjuvant.

In our work on transcutaneous immunisation that has been previously disclosed, we have immunised with a formulation in liquid form: as a solution, suspension, gel, emulsion, or liposome preparation. We have now discovered that a dry form of the formulation may be successfully used and, surprisingly, the immune response may be more robust than immunisation with liquid formulations.

These and other advantages of the invention are discussed below.

### BRIEF DESCRIPTION OF DRAWING

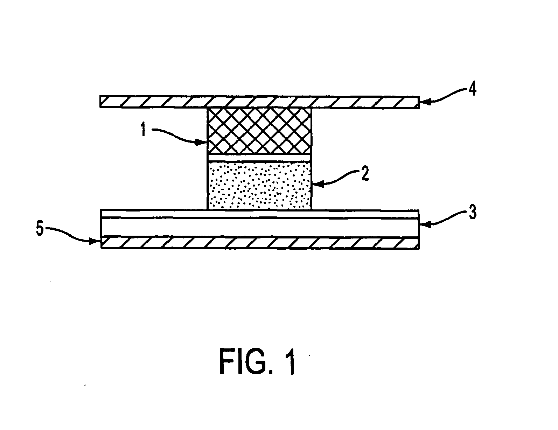

An exemplary patch of the invention is shown in Fig. 1. Forumulation of the invention may be applied to gauze (1) adhered to backing (2) as a circular unit of 1 cm² diameter. The circular unit is adhered to a rectangular dressing (3) to form a patch that can be applied to skin of a subject with the gauze-side in contact with the skin, adhered thereto by exposed adhesive, and covered by the dressing-side. The patch is packaged aseptically and can be stored at room temperature wrapped in release liner (4) on the gauze-side and carrier paper (5) on the dressing-side. Components can be held in close apposition by adhesive laminates, except for the wrappings of the patch. The thickness and other dimensions of the patch are not drawn to scale.

### DESCRIPTION OF THE INVENTION

An object of the invention is to provide an improved system for transcutaneous immunisation which induces an immune response (e.g. humoral and/or cellular effector) in an animal or human. The delivery system provides simple application of a formulation comprised of antigen and adjuvant or polynucleotide (encoding adjuvant or antigen) to the skin of a subject, thereby inducing a specific immune response against an antigen. Most importantly, at least one ingredient or component of the formulation (i.e., antigen or adjuvant) is provided in dry form prior to administration of the formulation.

For example, activation of antigen, adjuvant, or antigen-presenting cell may assist in the presentation of antigen by immune cells. Activation may promote contact between the formulation and an antigen presenting cell of the immune system (e.g., Langerhans cells in the epidermis, dermal dendritic cells, follicular dendritic cells, macrophages, B cells) and/or induce the antigen presenting cell to take up the formulation; the antigen presenting cell would then present antigen to a lymphocyte. In particular, the antigen presenting cell may migrate from the skin to the lymph nodes, and then present antigen to a lymphocyte, thereby inducing an antigen-specific immune response. Furthermore, the formulation may directly contact a lymphocyte which recognizes antigen, thereby inducing an antigen-specific immune response.

In addition to eliciting immune reactions leading to activation and/or expansion of an antigen-specific B and/or T cell population, including a cytotoxic T lymphocyte (CTL), another object of the invention is to positively and/or negatively regulate components of the immune system by using the transcutaneous immunization system to affect antigen-specific helper (Th1 and/or Th2) or delayed-type hypersensitivity (DTH) T-cell subsets. This can be exemplified by the differential behavior of CT and LT which can result in different T-helper responses. The desired immune response is preferably systemic or regional (e.g., mucosal), but not an allergic reaction, dermatitis, eczema, psoriasis, or other atopic reaction. It would be expected that these immune responses could lead to protective immune responses such as anti-tetanus toxoid antibodies for tetanus or anti-diphtheria antibodies for diphtheria.

The invention may be practiced without perforation of skin. For example, it may be practiced with chemical or physical penetration enhancement. For example, swabbing the skin with an aqueous solution (e.g., water, saline and buffered solutions), alcohol (e.g., isopropyl alcohol), polyethylene glycol, and glycerol, or incorporating same in the formulation, may act as a chemical penetration enhancer. Similarly, abrading the skin with an abrasive (e.g., emery board), or removing a superficial layer of skin by peeling (e.g., stripping off adhesive tape) may act as a physical penetration enhancer. See Walters and Hadgraft (1993) and WO 99/43350. Alternatively, applying the formulation to intact skin does not involve physical, electrical, or sonic energy is not involved to perforate layers of the skin below the stratum corneum.

Another object of the invention is to provide formulations useful for immunization and vaccination, as well as processes for their manufacture. A dry formulation is more easily stored and transported than conventional vaccines, it breaks the cold chain required from the vaccine's place of manufacture to the locale where vaccination occurs. Without being limited to any particular mode of action, another way in which a dry formulation may be an improvement over liquid formulations is that high concentrations of a dry active ingredient of the formulation (e.g., antigen and adjuvant) may be achieved by solubilization directly at the site of immunization over a short time span. Moisture from the skin and an occlusive dressing may hasten this process. In this way, it is possible that a concentration approaching the solubility limit of the active ingredient may be achieved in situ. Alternatively, the dry, active ingredient of the formulation per se may be the improvement by providing a solid particulate form that is taken up and processed by antigen presenting cells. These possible mechanisms are discussed not to limit the scope of the invention or its equivalents, but to provide insight into the operation of the invention and to guide the use of this formulation in immunization and vaccination.

The dry formulation of the invention may be provided in various forms. A "patch" refers to an embodiment which includes a solid substrate (e.g., medical dressing) as well as at least one active ingredient. Other embodiments are fine or granulated powders, dry uniform films, pellets, and tablets. The formulation may be dissolved, and then dried in an ampoule or on a flat surface (e.g., skin), or simply dusted on the flat surface. It may be air dried, dried with elevated temperature, freeze or spray dried, coated or sprayed on a solid substrate and then dried, dusted on a solid substrate, quick frozen and then slowly dried under vacuum, or combinations thereof. If different molecules are active ingredients of the formulation, they may be mixed in solution and then dried, or mixed in dry form only. Compartments or chambers of the patch may be used to separate active ingredients so that only one of the antigens or adjuvants is kept in dry form prior to administration; separating liquid and solid in this manner allows control over the time and rate of the dissolving of at least one dry, active ingredient. Optionally the formulation may include excipient, stabilizer, dessicant, preservative, adhesive, patch materials, or combinations thereof.

The formulation may be manufactured under aseptic conditions with practices acceptable to the appropriate regulatory agencies (e.g., the Food and Drug Administration) for biologicals and vaccines.

An embodiment of the invention is to provide a single or unit dose of formulation for administration. The amount of antigen or adjuvant in the unit dose may be anywhere in a broad range from about 0.1 µg to about 10 mg. The range from about 1 µg to about 1 mg is preferred, the range from about 10 µg to about 500 µg is more preferred. Other suitable ranges are between about 1 µg and about 10 µg, between about 10 µg and about 50 µg, between about 50 µg and about 200 µg, and between about 1 mg and about 5 mg. The ratio between antigen and adjuvant may be 1:1 (e.g., CT when it is both antigen and adjuvant) but higher ratios may be suitable for poor antigens, or lower ratios of adjuvant to antigen may be used.

Further embodiments of the invention are described below.

In one embodiment of the invention, a formulation comprising adjuvant and antigen is applied to skin of an infected subject, antigen is presented to immune cells, and an antigen-specific immune response is induced. The formulation may contain only antigen, but no additional adjuvant, if antigenicity of the formulation is sufficient to not require adjuvant activity. The formulation may include an additional antigen such that application of the formulation induces an immune response against multiple antigens). In such a case, the antigens may or may not be derived from the same source, but the antigens will have different chemical structures so as to induce immune responses specific for the different antigens. Antigen-specific lymphocytes may participate in the immune response and, in the case of participation by B lymphocytes, antigen-specific antibodies may be part of the immune response. The formulations described above may include excipients, stabilizers, dessicants, preservatives, adhesives, and patch materials known in the art.

In another embodiment of the invention, the invention is used to treat a subject. If the antigen is derived from a pathogen, the treatment vaccinates the subject against infection by the pathogen or against its pathogenic effects such as those caused by toxin secretion. A formulation that includes a tumor antigen may provide a cancer treatment; a formulation that includes an autoantigen may provide a treatment for a disease caused by the subject's own immune system (e.g., autoimmune disease). The invention may be used therapeutically to treat existing disease, protectively to prevent disease, to reduce the severity and/or duration of disease, or to ameliorate symptoms of disease.

In a further embodiment of the invention, a patch for use in the above methods is provided. The patch may comprise a dressing, and effective amounts of antigen and adjuvant. The dressing may be occlusive or non-occlusive.

A patch containing adjuvant and antigen may contain a single reservoir or multiple reservoirs with individual components of the formulation. The patch may include additional antigens such that application of the patch induces an immune response to multiple antigens. In such a case, the antigens may or may not be derived from the same source, but the antigens will have different chemical structures so as to induce an immune response specific for the different antigens. Multiple patches may be applied simultaneously; a single patch may contain multiple reservoirs. For effective treatment, multiple patches may be applied at frequent intervals or constantly over a period of time (see U.S. Pat. No. 5,049,387 and Examples for a detailed description of a patch); or may be applied simultaneously.

Formulation in liquid or solid form may be applied in a similar fashion using multiple antigens and adjuvants both at the same or separate sites or simultaneously or in frequent, repeated applications. The patch may include a controlled, released reservoir or, a matrix or rate controlling membrane may be used which allows stepped release of antigen. The patch may contain a single reservoir with either antigen or adjutant, or multiple reservoirs to separate individual antigens and adjuvants.

But at least one antigen or adjuvant must be maintained in dry form prior to administration. Subsequent release of liquid from a reservoir or entry of liquid into a reservoir containing the dry ingredient of the formulation will at least partially dissolve that ingredient.

The application site may be protected with anti-inflammatory corticosteroids such as hydrocortisone, triamcinolone and mometazone or non-steroidal anti-inflammatory drugs (NSAIDs) to reduce possible local skin reaction or modulate the type of immune response. Similarly, anti-inflammatory steroids or NSAIDs may be included in the patch material, in creams, ointments, etc. and corticosteroids or NSAIDs may be applied after immunization. IL-10, TNF-α, other immunomodulators may be used instead of the anti-inflammatory agents. Moreover, in yet another embodiment of the invention, the formulation is applied to intact skin overlying more than one draining lymph node field using either single or multiple applications. The formulation may include additional antigens such that application to intact skin induces an immune response to multiple antigens. In such a case, the antigens may or may not be derived from the same source, but the antigens will have different chemical structures so as to induce an immune response specific for the different antigens. Multi-chambered patches could allow more effective delivery of multivalent vaccines as each chamber covers an individual set of antigen presenting cells. Thus, antigen presenting cells would encounter only one antigen (plus adjuvant) and thus would eliminate antigenic competition and thereby enhancing the response to each individual antigen in the multivalent vaccine.

The formulation may be applied to intact skin to boost or prime the immune response in conjunction with physically penetrating or other routes of immunization. Thus, priming with transcutaneous immunization with either single or multiple applications may be followed with oral, nasal, or parenteral techniques for boosting immunization with the same or altered antigens. The formulation may include additional antigens such that application to intact skin induces an immune response to multiple antigens.

In addition to antigen and adjuvant, the formulation may comprise a vehicle. For example, the formulation may comprise AQUAPHOR (an emulsion of petrolatum, mineral oil, mineral wax, wool wax, panthenol, bisabol, and glycerin as shown in WO 98/20734), emulsions (e.g., aqueous creams), microemulsions, gels, oil-in-water emulsions (e.g., oily creams), anhydrous lipids and oil-in-water emulsions, other types of emulsions, fats, waxes, oil, silicones, gels and humectants (e.g., glycerol). But at least one antigen or adjuvant is maintained in dry form prior to administration.

The antigen may be derived from a pathogen that can infect the subject (e.g., bacterium, virus, fungus, or parasite), or a cell (e.g., tumor cell or normal cell). The antigen may be a tumor antigen or an autoantigen. The antigen may be an allergen such as pollen, animal dander, mold, dust mite, flea allergen, salivary allergen, grass, food (e.g., peanuts and other nuts), Bet v1 (Wiedermann et al., 1998), or even a contact sensitizer like nickel or DNCB. The autoantigen may be associated with autoimmune disease such as an islet antigen (Ramiya et al., 1996). Chemically, the antigen may be a carbohydrate, glycolipid, glycoprotein, lipid, lipoprotein, phospholipid, polypeptide, or chemical or recombinant conjugate of the above. The molecular weight of the antigen may be greater than 500 daltons, preferably greater than 800 daltons, and more preferably greater than 1000 daltons.

Antigen may be obtained by recombinant techniques, chemical synthesis, or purification from a natural source. The antigen can be proteinaceous or a conjugate with polysaccharide. Antigen may be provided as live virus, attenuated live virus, or virus that has been inactivated by chemical or genetic techniques. Alternatively, antigen may be at least partially purified in cell-free form (e.g., membrane fraction, whole cell lysate).

Inclusion of an adjuvant may allow potentiation or modulation of the immune response. Moreover, selection of a suitable antigen or adjuvant may allow preferential induction of a humoral or cellular immune response, specific antibody isotypes (e.g., IgM, IgD, IgA1, IgA2, IgE, IgG1, IgG2, IgG3, and/or IgG4), and/or specific T-cell subsets (e.g., CTL, Th1, Th2 and/or T_{DTH}). Preferably the adjuvant is an activated ADP-ribosylating exotoxin or a subunit thereof.

The term "antigen" as used in the invention, is meant to describe a substance that induces a specific immune response when presented to immune cells of a subject. An antigen may comprise a single immunogenic epitope, or a multiplicity of immunogenic epitopes recognized by a B-cell receptor (i.e., antibody on the membrane of the B cell) or a T-cell receptor. A molecule may be both an antigen and an adjuvant (e.g., cholera toxin) and, thus, the formulation may contain only one ingredient or component.

The term "adjuvant" as used in the invention, is meant to describe a substance added to the formulation to assist in inducing an immune response to the antigen.

The term "effective amount" as used in the invention, is meant to describe that amount of antigen which induces an antigen-specific immune response.

Such induction of an immune response may provide a treatment such as, for example, immunoprotection, desensitization, immunosuppression, modulation of autoimmune disease, potentiation of cancer immunosurveillance, or therapeutic vaccination against an established infectious disease. A product or method "induces" when its presence or absences causes a statistically significant increase or decrease, respectively, in the immune response's magnitude and/or kinetics; change in the induced elements of the immune system (e.g., humoral vs. cellular, Th1 vs. Th2); effect on the health and well-being of the subject; or combinations thereof.

The term "draining lymph node field" as used in the invention means an anatomic area over which the lymph collected is filtered through a set of defined lymph nodes (e.g., cervical, axillary, inguinal, epitrochelear, popliteal, those of the abdomen and thorax).

Without being bound to any particular theory but only to provide an explanation for our observations, it is presumed that the transcutaneous immunization delivery system carries antigen to cells of the immune system where an immune response is induced. The antigen may pass through the normal protective outer layers of the skin (i.e., the stratum corneum) and induce the immune response directly, or through an antigen presenting cell population in the epidermis (e.g., macrophage, tissue macrophage, Langerhans cell, dendritic cell, dermal dendritic cell, B lymphocyte, or Kupffer cell) that presents processed antigen to a lymphocyte. Optionally, the antigen may pass through the stratum corneum via a hair follicle or a skin organelle (e.g., sweat gland, oil gland).

For example, transcutaneous immunization with bacterial ADP-ribosylating exotoxins (bAREs) as an example, may target the epidermal Langerhans cell, known to be among the most efficient of the antigen presenting cells (APCs). We have found that bAREs activate Langerhans cells when applied epicutaneously to intact skin. Adjuvants such as trypsin cleaved LT may enhance Langerhans cell activation. The Langerhans cells direct specific immune responses through phagocytosis of the antigens, and migration to the lymph nodes where they act as APCs to present the antigen to lymphocytes, and thereby induce a potent antibody response. Although the skin is generally considered a barrier to invading organisms, the imperfection of this barrier is attested to by the numerous Langerhans cells distributed throughout the epidermis that are designed to orchestrate the immune response against organisms invading via the skin. According to Udey (1997):
"Langerhans cells are bone-marrow derived cells that are present in all mammalian stratified squamous epithelia. They comprise all of the accessory cell activity that is present in uninflamed epidermis, and in the current paradigm are essential for the initiation and propagation of immune responses directed against epicutaneously applied antigens. Langerhans cells are members of a family of potent accessory cells ('dendritic cells') that are widely distributed, but infrequently represented, in epithelia and solid organs as well as in lymphoid tissue.
"It is now recognized that Langerhans cells (and presumably other dendritic cells) have a life cycle with at least two distinct stages. Langerhans cells that are located in epidermis constitute a regular network of antigen-trapping 'sentinel' cells. Epidermal Langerhans cells can ingest particulates, including microorganisms, and are efficient processors of complex antigens. However, they express only low levels of MHC class I and II antigens and costimulatory molecules (ICAM-1, B7-1 and B7-2) and are poor stimulators of unprimed T cells. After contact with antigen, some Langerhans cells become activated, exit the epidermis and migrate to T-cell-dependent regions of regional lymph nodes where they localize as mature dendritic cells. In the course of exiting the epidermis and migrating to lymph nodes, antigen-bearing epidermal Langerhans cells (now the 'messengers') exhibit dramatic changes in morphology, surface phenotype and function. In contrast to epidermal Langerhans cells, lymphoid dendritic cells are essentially non-phagocytic and process protein antigens inefficiently, but express high levels ofMHC class I and class II antigens and various costimulatory molecules and are the most potent stimulators of naive T cells that have been identified."

We envision that the potent antigen presenting capability of the epidermal Langerhans cells can be exploited for transcutaneously delivered vaccines. A transcutaneous immune response using the skin immune system may be achieved by delivering the formulation only to Langerhans cells in the stratum corneum (i.e., the outermost layer of the skin consisting of cornified cells and lipids) by, for example, passive diffusion and subsequent activation of the Langerhans cells to take up antigen, migrate to B-cell follicles and/or T-cell dependent regions, and present the antigen to B and/or T cells. If antigens other that bAREs (e.g., BSA) are to be phagocytosed by the Langerhans cells, then these antigens could also be taken to the lymph node for presentation to T-cells and subsequently induce an immune response specific for that antigen (e.g., BSA). Thus, a feature of transcutaneous immunization is the activation of the Langerhans cell, presumably by a bAREs, or by trypsin-activated LT or other activated bacterial ADP-ribosylating exotoxins, ADP-ribosylating exotoxin binding subunits (e.g., cholera toxin B subunit), recombinant fusion proteins, cytokines such as TNFα, IL-1β, active peptide fragments of IL1β, LPS, LPS derivatives and analogs, lipid A or other Langerhans cell activating substance including contact sensitizers or adjuvants. Increasing the size of the skin population of Langerhans cells or their state of activation through swabbing or acetone treatment would also be expected to enhance the immune response. In aged or LC-depleted skin (i.e., from UV damage) it may be possible to use tretinoin to replenish the population of Langerhans cells (Murphy et al., 1998).

It should be noted that adjuvants such as LPS are known to be highly toxic when injected or given systemically (Rietschel et al., 1994; Vosika et al., 1984) but if placed on the surface of intact skin are unlikely to induce systemic toxicity and thus the transcutaneous route may allow the advantage of adjuvant effects without systemic toxicity, similar to our findings with CT. A similar absence of toxicity could be expected if the skin were penetrated only below the stratum corneum. Thus, the ability to induce activation of the immune system through the skin confers the unexpected advantage of potent immune responses without systemic toxicity.

In addition, the magnitude of the antibody response induced by transcutaneous immunization and isotype switching to predominantly IgG is generally achieved with T-cell help (Janeway and Travers, 1996), and activation of both Th1 and Th2 pathways is suggested by the production of IgG1 and IgG2a (Paul and Seder, 1994; Seder and Paul, 1994). Alternatively, a large antibody response may be induced by a thymus-independent antigen type 1 (TI-1) which directly activates the B cell (Janeway and Travers, 1996) or could have similar activating effects on B-cells such as up-regulation of MHC Class II, B7, CD40, CD25, and ICAM-1 (Nashar et al., 1997).

The spectrum of more commonly known skin immune responses is represented by contact dermatitis and atopy. Contact dermatitis, a pathogenic manifestation of LC activation, is directed by Langerhans cells which phagocytose antigen, migrate to lymph nodes, present antigen, and sensitize T cells that migrate to the skin and cause the intense destructive cellular response that occurs at affected skin sites (Dahl, 1996; Leung, 1997). Such responses are not generally known to be associated with antigen specific IgG antibodies. Atopic dermatitis may utilize the Langerhans cell in a similar fashion, but is identified with Th2 cells and is generally associated with high levels of IgE antibody (Dahl, 1996; Leung, 1997).

Transcutaneous immunization with cholera toxin and related bAREs on the other hand is a novel immune response with an absence of findings typical of atopy or contact dermatitis given the high levels of IgG that are induced. For example, application of cholera toxin epicutaneously to intact skin results achieves immunization in the absence of lymphocyte infiltration 24, 48 and 120 hours after immunization. This indicates that Langerhans cells engaged by transcutaneous immunization as they "comprise all of the accessory cell activity that is present in uninflamed epidermis, and in the current paradigm are essential for the initiation and propagation of immune responses directed against epicutaneously applied antigens" (Udey, 1997). The uniqueness of the transcutaneous immune response here is also indicated by the both high levels of antigen-specific IgG antibody, and the type of antibody produced (e.g., IgM, IgG1, IgG2a, IgG2b, IgG3 and IgA) and generally the absence of antigen specific IgE antibody. Transcutaneous immunization could conceivably occur in tandem with skin inflammation if sufficient activation of APCs and T-cells were to occur in a transcutaneous response coexisting with atopy or contact dermatitis.

Transcutaneous targeting of Langerhans cells may also be used in tandem with agents to deactivate all or part of their antigen presenting function, thereby modifying immunization or preventing sensitization. Techniques to modulate Langerhans activation or other skin immune cells include, for example, the use of anti-inflammatory steroidal or non-steroidal agents (NSAID), cyclophosphamide or other immunosuppressants, interleukin-10, monoclonal antibody to interleukin-1, interleukin-1 converting enzyme (ICE) inhibitors, interleukin-1 receptor antagonist (RA), or depletion via superantigens such as through staphylococcal enterotoxin-A (SEA) induced epidermal Langerhans cell depletion. Similar compounds may be used to modify the innate response of Langerhans cells and induce different T-helper responses (Th1 or Th2) or may modulate skin inflammatory responses to decrease potential side effects of the immunization.

Similarly, lymphocytes may be immunosuppressed before, during or after immunization by administering immunosupressant separately or by co-administrating immunosupressant with the formulation. For example, it may be possible to induce a potent systemic protective immune responses with agents that would normally result in allergic or irritant contact hypersensitivity but adding inhibitors of ICE may alleviate adverse skin reactions (Zepter et al., 1997).

Transcutaneous immunization may be induced via the ganglioside GM1 binding activity of CT, LT or subunits such as CTB. Ganglioside GM1 is a ubiquitous cell membrane glycolipid found in all mammalian cells. When the pentameric CT B subunit binds to the cell surface, a hydrophilic pore is formed which allows the A subunit to insert across the lipid bilayer (Ribi et al., 1988). Other binding targets on the APCs may be utilized. The B-subunit of LT binds to ganglioside GM1 in addition to other gangliosides and its binding activities may account for its the fact that LT is highly immunogenic on the skin.

Transcutaneous immunization by CT or CTB may require ganglioside GM1 binding activity. When mice are transcutaneously immunized with CT, CTA and CTB, only CT and CTB resulted in an immune response. CTA contains the ADP-ribosylating exotoxin activity but only CT and CTB containing the binding activity are able to induce an immune response indicating that the B subunit was necessary and sufficient to immunize through the skin. We conclude that the Langerhans cells or other APCs may be activated by CTB binding to its cell surface resulting in a transcutaneous immune response.

### ANTIGEN

A transcutaneous immunization system delivers agents to specialized cells (e.g., antigen presentation cell, lymphocyte) that produce an immune response. These agents as a class are called antigens. Antigen may be composed of chemicals such as, for example, carbohydrate, glycolipid, glycoprotein, lipid, lipoprotein, phospholipid, polypeptide, conjugates thereof, or any other material known to induce an immune response. Antigen may be provided as a whole organism such as, for example, a bacterium or virion; antigen may be obtained from an extract or lysate, either from whole cells or membrane alone; or antigen may be chemically synthesized or produced by recombinant means.

Antigen of the invention may be expressed by recombinant means, preferably as a fusion with an affinity or epitope tag (Summers and Smith, 1987; Goeddel, 1990; Ausubel et al., 1996); chemical synthesis ofan oligopeptide, either free or conjugated to carrier proteins, may be used to obtain antigen of the invention (Bodanszky, 1993; Wisdom, 1994). Oligopeptides are considered a type of polypeptide. Oligopeptide lengths of 6 residues to 20 residues are preferred. Polypeptides may also by synthesized as branched structures such as those disclosed in U.S. Pat. Nos. 5,229,490 and 5,390,111. Antigenic polypeptides include, for example, synthetic or recombinant B-cell and T-cell epitopes, universal T-cell epitopes, and mixed T-cell epitopes from one organism or disease and B-cell epitopes from another. Antigen obtained through recombinant means or peptide synthesis, as well as antigen of the invention obtained from natural sources or extracts, may be purified by means of the antigen's physical and chemical characteristics, preferably by fractionation or chromatography (Janson and Ryden, 1997; Deutscher, 1990; Scopes, 1993). Recombinants may combine B subunits or chimeras of bAREs (Lu et al., 1997). A multivalent antigen formulation may be used to induce an immune response to more than one antigen at the same time. Conjugates may be used to induce an immune response to multiple antigens, to boost the immune response, or both. Additionally, toxins may be boosted by the use of toxoids, or toxoids boosted by the use of toxins. Transcutaneous immunization may be used to boost responses induced initially by other routes of immunization such as by oral, nasal or parenteral routes. Antigen includes, for example, toxins, toxoids, subunits thereof, or combinations thereof (e.g., cholera toxin, tetanus toxoid); additionally, toxins, toxoids, subunits thereof, or combinations thereof may act as both antigen and adjuvant. Such oral/transcutaneous or transcutaneous/oral immunization may be especially important to enhance mucosal immunity in diseases where mucosal immunity correlates with protection.

Antigen may be solubilized in a buffer or water or organic solvents such as alcohol or DMSO, or incorporated in gels, emulsion, microemulsions, and creams. Suitable buffers include, but are not limited to, phosphate buffered saline Ca⁺⁺/Mg⁺⁺ free (PBS), phosphate buffered saline (PBS), normal saline (150 mM NaCl in water), and Tris buffer. Antigen not soluble in neutral buffer can be solubilized in 10 mM acetic acid and then diluted to the desired volume with a neutral buffer such as PBS. In the case of antigen soluble only at acid pH, acetate-PBS at acid pH may be used as a diluent after solubilization in dilute acetic acid. Glycerol may be a suitable non-aqueous buffer for use in the invention.

Hydrophobic antigen can be solubilized in a detergent, for example a polypeptide containing a membrane-spanning domain. Furthermore, for formulations containing liposomes, an antigen in a detergent solution (e.g., a cell membrane extract) may be mixed with lipids, and liposomes then may be formed by removal of the detergent by dilution, dialysis, or column chromatography, see, Gregoriadis (1995). Certain antigens such as, for example, those from a virus (e.g., hepatitis A) need not be soluble per se, but can be incorporated directly into a lipid membrane (e.g., a virosome as described by Morein and Simons, 1985), in a suspension of virion alone, or suspensions of microspheres or heat-inactivated bacteria which may be taken up by activate antigen presenting cells (e.g., opsonization). Antigens may also be mixed with a penetration enhancer as described in WO 99/43350. Plotkin and Mortimer(1994) provide antigens which can be used to vaccinate animals or humans to induce an immune response specific for particular pathogens, as well as methods of preparing antigen, determining a suitable dose of antigen, assaying for induction of an immune response, and treating infection by a pathogen (e.g., bacterium, virus, fungus, or parasite).

Bacteria include, for example: anthrax, campylobacter, cholera, clostridia including clostridium difficile, diphtheria, enterohemorrhagic E. coli, enterotoxigenic E. coli, giardia, gonococcus, Helicobacter pylori or urease produced by H. pylori (Lee and Chen, 1994), Hemophilus influenza B, Hemophilus influenza non-typable, Legionella, meningococcus, mycobacterium including those organisms responsible for tuberculosis, pertussis, pneumococcus, salmonella, shigella, staphylococcus and their enterotoxins, Group A beta-hemolytic streptococcus, Streptococcus B, tetanus, Vibrio cholerae, Borrelia burgdorfi and Yersinia; and products thereof.

Viruses include, for example: adenovirus, dengue serotypes 1 to 4 (Delenda et al., 1994; Fonseca et al., 1994; Smucny et al., 1995), ebola (Jahrling et al., 1996), enterovirus, hanta virus, hepatitis serotypes A to E (Blum, 1995; Katkov, 1996; Lieberman and Greenberg, 1996; Mast, 1996; Shafara et al., 1995; Smedila et al., 1994; U.S. Pat. Nos. 5,314,808 and 5,436,126), herpes simplex virus 1 or 2, human immunodeficiency virus (Deprez et al., 1996), human papilloma virus, influenza, measles, Norwalk, Japanese equine encephalitis, papilloma virus, parvovirus B19, polio, rabies, respiratory syncytial virus, rotavirus, rubella, rubeola, St. Louis encephalitis, vaccinia, viral expression vectors containing genes coding for other antigens such as malaria antigens, varicella, and yellow fever; and products thereof.

Parasites include, for example: Entamoeba histolytica (Zhang et al., 1995); Plasmodium (Bathurst et al., 1993; Chang et al., 1989, 1992, 1994; Fries et al., 1992a, 1992b; Herrington et al., 1991; Khusmith et al., 1991; Malik et al., 1991; Migliorini et al., 1993; Pessi et al., 1991; Tam, 1988; Vreden et al., 1991; White et al., 1993; Wiesmueller et al., 1991), Leishmania (Frankenburg et al., 1996), and the Helminthes; Schistosomes; and products thereof.

Fungi including entities responsible for tinea corporis_{,} tinea unguis, sporotrichosis, aspergillosis, candida and other pathogenic fungi.

### ADJUVANT

The formulation also contains an adjuvant, although a single molecule may contain both adjuvant and antigen properties (e.g., cholera toxin) (Elson and Dertzbaugh, 1994). Adjuvants are substances that are used to specifically or non-specifically potentiate an antigen-specific immune response. Usually, the adjuvant and the formulation are mixed prior to presentation of the antigen but, alternatively, they may be separately presented within a short interval of time.

The adjuvant in the formulation of the invention comprises a bacterial ADP-ribosylating exotoxin or λ-subunit thereof, which encompasses a ganglioside GM1-binding, mutant bacterial ADP-ribosylating exotoxins including mutants at the tyrpsin cleavage site (Dickenson and Clements, 1995) and affecting ADP-ribosylation (Douce et al., 1997). Other adjuvants include, for example, an oil emulsion (e.g., complete or incomplete Freund's adjuvant), a chemokine (e.g., defensins 1 or 2, RANTES, MIP1-α, MIP-2, interleukin-8, or a cytokine (e.g., interleukin-1β, -2, -6, -10 or -12; interferon gamma; tumor necrosis factor-α; or granulocyte-monocyte-colony stimulating factor) (reviewed in Nohria and Rubin, 1994), a muramyl dipeptide derivative (e.g., murabutide, threonyl-MDP or muramyl tripeptide), a heat shock protein or a derivative, a derivative of Leishmania major LeIF (Skeiky et al.,1995), a lipopolysaccharide (LPS) derivative (e.g., lipid A or monophosphoryl lipid A or lipid A analogs), superantigen (Saloga et al., 1996b), QS21, Quill A or alum. Also, see Richards et al. (1995) for other adjuvants useful in immunization.

An adjuvant may be chosen to preferentially induce antibody or cellular effectors, specific antibody isotypes (e.g., IgM, IgD, IgA1, IgA2, secretory IgA, IgE, IgG1, IgG2, IgG3, and/or IgG4), or specific T-cell subsets (e.g., CTL, Th1, Th2 and/or T_{DTH}) (see, for example, Munoz et al., 1990; Glenn et al., 1995).

Unmethylated CpG dinucleotides or motifs are known to activate B cells and macrophages (Kreig et al., 1995; Stacey et al., 1996). Other forms of bacterial DNA can be used as adjuvants. Bacterial DNAs are among a class of structures which have patterns allowing the immune system to recognize their pathogenic origins to stimulate the innate immune response leading to adaptive immune responses (Medzhitov and Janeway, 1997). These structures are called pathogen-associated molecular patterns (PAMPs) and include lipopolysaccharides, teichoic acids, unmethylated CpG motifs, double-stranded RNA, and mannins. PAMPs induce endogenous signals that can mediate the inflammatory response, act as co-stimulators of T-cell function and control the effector function. The ability of PAMPs to induce these responses play a role in their potential as adjuvants and their targets are APCs such as macrophages and dendritic cells. The antigen presenting cells of the skin could likewise be stimulated by PAMPs transmitted through the skin. For example, Langerhans cells, a type of dendritic cell, could be activated by a PAMP in solution on the skin with a transcutaneously poorly immunogenic molecule and be induced to migrate and present this poorly immunogenic molecule to T-cells in the lymph node, inducing an antibody response to the poorly immunogenic molecule. PAMPs could also be used in conjunction with other skin adjuvants such as cholera toxin to induce different co-stimulatory molecules and control different effector functions to guide the immune response, for example from a Th2 to a Th1 response.

Cholera toxin is a bacterial exotoxin from the family of ADP-ribsoylating exotoxins (referred to as bAREs). Most bAREs are organized as A:B dimer with a binding B subunit and an A subunit containing the ADP-ribosyltransferase. Such toxins include diphtheria, Pseudomonas exotoxin A, cholera toxin (CT), E. coli heat-labile enterotoxin (LT), pertussis toxin, C. botulinum toxin C2, C. botulinum toxin C3, C. limosum exoenzyme, B. cereus exoenzyme, Pseudomonas exotoxin S, Staphylococcus aureus EDIN, and B. sphaericus toxin.

Cholera toxin is an example of a bARE that is organized with A and B subunits. The B subunit is the binding subunit and is non-covalently bound to the A subunit. The B subunit pentamer is arranged in a symmetrical doughnut-shaped structure that binds to GM1-ganglioside on the target cell. The A subunit serves to ADP ribosylate the alpha subunit of a subset of the heterotrimeric GTP proteins (G proteins) including the Gs protein which results in the elevated intracellular levels of cyclic AMP. This stimulates release of ions and fluid from intestinal cells in the case of cholera. However, cholera toxin (CT) and its B subunit (CTB) have adjuvant properties when used as either an intramuscular or oral immunogen (Elson and Dertzbaugh, 1994; Trach et al., 1997). Heat-labile enterotoxin from E. coli (LT) is 75-77% homologous at the amino acid level with CT and possesses similar binding properties; it also appears to bind the GM1-ganglioside receptor in the gut and has similar ADP-ribosylating exotoxin activities. Pseudomonas exotoxin A (ETA) binds to the α2-macroglobulin receptor-low density lipoprotein receptor-related protein (Kounnas et al., 1992). bAREs are reviewed by Krueger and Barbieri (1995). CT, CTB, LT, ETA and PT, despite having different cellular binding sites, are potent adjuvants for transcutaneous immunization, inducing IgG antibodies but not IgE antibodies. CTB without CT can also induce IgG antibodies. Thus, both bAREs and a derivative thereof can effectively immunize when epicutaneously applied to the skin in a simple solution.

When an adjuvant such as CT is mixed with BSA, a protein not usually immunogenic when applied to the skin, anti-BSA antibodies are induced. An immune response to diphtheria toxoid was induced using pertussis toxin as adjuvant, but not with diphtheria toxoid alone. Native LT as an adjuvant and antigen, however, is clearly not as potent as native CT. But activated bAREs can act as adjuvants for non-immunogenic proteins in an transcutaneous immunization system. Thus, therapeutic immunization with an antigen for the organism such as HIV, HPV or leishmania could be used separately or in conjunction with immunostimulation of the infected APCs to induce a therapeutic immunization.

Protection against the life-threatening infections diphtheria, pertussis, and tetanus (DPT) can be achieved by inducing high levels of circulating anti-toxin antibodies. Pertussis may be an exception in that some investigators feel that antibodies directed to other portions of the invading organism are necessary for protection, although this is controversial (see Schneerson et al., 1996) and most new generation acellular pertussis vaccines have PT or genetically detoxified PT as a component of the vaccine (Krueger and Barbieri, 1995). The pathologies in the diseases caused by DPT are directly related to the effects of their toxins and anti-toxin antibodies most certainly play a role in protection (Schneerson et al., 1996).

In general, toxins can be chemically inactivated to form toxoids which are less toxic but remain immunogenic. We envision that the transcutaneous immunization system using toxin-based immunogens and adjuvants can achieve anti-toxin levels adequate for protection against these diseases. The anti-toxin antibodies may be induced through immunization with the toxins, or genetically-detoxified toxoids themselves, or with toxoids and adjuvants such as CT. Genetically toxoided toxins which have altered ADP-ribosylating exotoxin activity or trypsin cleavage site, but not binding activity, are envisioned to be especially useful as non-toxic activators of antigen presenting cells used in transcutaneous immunization and may reduce concerns over toxin use.

CT can also act as an adjuvant to induce antigen-specific CTLs through transcutaneous immunization. The bARE adjuvant may be chemically conjugated to other antigens including, for example, carbohydrates, polypeptides, glycolipids, and glycoprotein antigens. Chemical conjugation with toxins, their subunits, or toxoids with these antigens would be expected to enhance the immune response to these antigens when applied epicutaneously. To overcome the problem of the toxicity of the toxins, (e.g., diphtheria toxin is known to be so toxic that one molecule can kill a cell) and to overcome the difficulty of working with such potent toxins as tetanus, several workers have taken a recombinant approach to producing genetically produced toxoids. This is based on inactivating the catalytic activity of the ADP-ribosyl transferase by genetic deletion. These toxins retain the binding capabilities, but lack the toxicity, of the natural toxins. This approach is described by Burnette et al. (1994), Rappuoli et al. (1995), and Rappuoli et al. (1996). Such genetically toxoided exotoxins would be expected to induce a transcutaneous immune response and to act as adjuvants (Douce et al., 1997). They may provide an advantage in a transcutaneous immunization system in that they would not create a safety concern as the toxoids would not be considered toxic. Activation through a technique such as trypsin cleavage, however, would be expected to enhance the adjuvant qualities of LT through the skin which is lacking inherent trypsin enzymes. Additionally, several techniques exist to chemically toxoid toxins which can address the same problem (Schneerson et al., 1996). These techniques could be important for certain applications, especially pediatric applications, in which ingested toxins (e.g., diphtheria toxin) might possibly create adverse reactions.

Optionally, an activator of Langerhans cells may be present in the formulation. Examples of such activators include: an inducer of heat shock protein; contact sensitizer (e.g., trinitrochlorobenzene, dinitrofluorobenzene, nitrogen mustard, pentadecylcatechol); toxin (e.g., Shiga toxin, Staph enterotoxin B); lipopolysaccharide, lipid A, or derivatives thereof; bacterial DNA (Stacey et al., 1996); cytokine (e.g., tumor necrosis factor-α, interleukin-1β -10, -12); a member of the TGFβ superfamily, calcium ions in solution, calcium ionophore, and a chemokine (e.g., defensins 1 or 2, RANTES, MIP-1α, MIP-2, interleukin-8).

In addition, formulations containing a small amount of cholera toxin along with at least one of IL-1β, IL-1α, TNF-α, CTB and LPS are envisioned. The molar ratio of the two parts of the formulation may be between about 0.001 and about 0.20.

If an immunizing antigen has sufficient Langerhans cell activating capabilities then a separate adjuvant may not be required, as in the case of CT which is both antigen and adjuvant. Alternatively, such antigens can be considered not to require an adjuvant to induce an immune response because they are sufficiently immunogenic. It is envisioned that whole cell preparations, live viruses, attenuated viruses, DNA plasmids, and bacterial DNA could be sufficient to immunize transcutaneously. It may also be possible to use low concentrations of contact sensitizers or other activators of Langerhans cells to induce an immune response without inducing skin lesions.

### FORMULATION

Processes for manufacturing a pharmaceutical formulation are well known. The components of the formulation may be combined with a pharmaceutically-acceptable carrier or vehicle, as well as any combination of optional additives (e.g., diluents, binders, excipients, stabilizers, dessicants, preservatives, colorings). The use of solid carriers, and the addition of excipients to assist in solubilization of dry components or stabilizers of antigenic or adjuvant activity, are preferred embodiments. See, generally, Ullmann's Encyclopedia of Industrial Chemistry, 6th Ed. (electronic edition, 1998); Remington's Pharmaceutical Sciences, 22nd (Gennaro, 1990, Mack Publishing); Pharmaceutical Dosage Forms, 2nd Ed. (various editors, 1989-1998, Marcel Dekker); and Pharmaceutical Dosage Forms and Drug Delivery Systems (Ansel et al., 1994, Williams & Wilkins).

Good manufacturing practices are known in the pharmaceutical industry and regulated by government agencies (e.g., Food and Drug Administration). Sterile liquid formulations may be prepared by dissolving an intended component of the formulation in a sufficient amount of an appropriate solvent, followed by sterilization by filtration to remove contaminating microbes. Generally, dispersions are prepared by incorporating the various sterilized components of the formulation into a sterile vehicle which contains the basic dispersion medium. For production of solid forms that are required to be sterile, vacuum drying or freeze drying can be used.

In general, solid dosage forms (e.g., powders, granules, pellets, tablets) can be made from at least one active ingredient or component of the formulation.

Suitable tableting procedures and the production of patches are known. The formulation may also be produced by encapsulating solid forms of at least one active ingredient, or keeping them separate from liquids in compartments or chambers. In one embodiment, the patch may include a pouch containing a vehicle (e.g., a saline solution) which is disrupted by pressure and subsequently solubilizes the dry formulation of the patch. The size of each dose and the interval of dosing to the subject may be used to determine a suitable size and shape of the tablet, capsule, compartment, or chamber.

Formulations will contain an effective amount of the active ingredients (e.g., antigen and adjuvant) together with carrier or suitable amounts of vehicle in order to provide pharmaceutically-acceptable compositions suitable for administration to a human or animal. Formulation that include a vehicle may be in the form of an cream, emulsion, gel, lotion, ointment, paste, solution, suspension, or other liquid forms known in the art; especially those that enhance skin hydration. For the invention, however, it is preferred that at least one active component of the formulation is in solid form.

The relative amounts of active ingredients within a dose and the dosing schedule may be adjusted appropriately for efficacious administration to a subject (e.g., animal or human). This adjustment may also depend on the subject's particular disease or condition, and whether treatment or prophylaxis is intended. To simplify administration of the formulation to the subject, each unit dose contains the active ingredients in predetermined amounts for a single round of immunization.

There are numerous causes of polypeptide instability or degradation, including hydrolysis and denaturation. In the case of denaturation, the conformation or three-dimensional structure of the protein is disturbed and the protein unfolds from its usual globular structure. Rather than refolding to its natural conformation, hydrophobic interaction may cause clumping of molecules together (i.e., aggregation) or refolding to an unnatural conformation. Either of these results may entail diminution or loss of antigenic or adjuvant activity. Stabilizers may be added to lessen or prevent such problems.

The formulation, or any intermediate in its production, may be pretreated with protective agents (i.e., cryoprotectants and dry stabilizers) and then subjected to cooling rates and final temperatures that minimize ice crystal formation. By proper selection of cryoprotective agents and use of pre-selected drying parameters, almost any formulation might be cryoprepared for a suitable desired end use.

It should be understood in the following discussion of optional additives like excipients, stabilizers, dessicants, and preservatives are described by their function. Thus, a particular chemical may act as some combination of excipient, stabilizer, dessicant, and/or preservative. Such chemical would be immunologically-inactive because it does not directly induce an immune response, but it increases the response by enhancing immunological activity of the antigen or adjuvant: for example, by reducing modification of the antigen or adjuvant, or denaturation during drying and dissolving cycles.

Stabilizers include cyclodextrin and derivatives thereof (see U.S. Pat. No. 5,730,969). Suitable preservatives such as sucrose, mannitol, sorbitol, trehalose, dextran, and glycerin can also be added to stabilize the final formulation (Howell and Miller, 1983). A stabilizer selected from nonionic surfactants, D-glucose, D-galactose, D-xylose, D-glucuronic acid, salts of D-glucuronic acid, trehalose, dextrans, hydroxyethyl starches, and mixtures thereof may be added to the formulation. Addition of an alkali metal salt or magnesium chloride may stabilize a polypeptide, optionally including serum albumin and freeze-drying to further enhance stability. A polypeptide may also be stabilized by contacting it with a saccharide selected from the group consisting of dextran, chondroitin sulfuric acid, starch, glycogen, insulin, dextrin, and alginic acid salt. Other sugars that can be added include monosaccharides, disaccharides, sugar alcohols, and mixtures thereof (e.g., glucose, mannose, galactose, fructose, sucrose, maltose, lactose, mannitol, xylitol). Polyols may stabilize a polypeptide, and are water-miscible or water-soluble. Suitable polyols may be polyhydroxy alcohols, monosaccharides and disaccharides including mannitol, glycerol, ethylene glycol, propylene glycol, trimethyl glycol, vinyl pyrrolidone, glucose, fructose, arabinose, mannose, maltose, sucrose, and polymers thereof. See, for example, Hanson and Roun (1992). Various excipients may also stabilize polpeptides, including serum albumin, amino acids, heparin, fatty acids and phospholipids, surfactants, metals, polyols, reducing agents, metal chelating agents, polyvinyl pyrrolidone, hydrolyzed gelatin, and ammonium sulfate.

Single-dose tetanus vaccine can be stabilized in poly(lactic acid) (PLA) and poly(lactide-co-glycolide) (PLGA) microspheres by suitable choice of excipient or stabilizer (Sanchez et al., 1999). Trehalose may be advantageously used as an additive because it is a non-reducing saccharide, and therefore does not cause aminocarbonyl reactions with substances bearing amino groups such as proteins.

It is conceivable that a formulation that can be administered to the subject in a dry, non-liquid form, may allow storage in conditions that do not require a cold chain. In one embodiment, an antigen in solution such as diphtheria toxoid is mixed in solution with an adjuvant such as CT and is placed on a gauze pad with an occlusive backing such as plastic wrap (Saran) and allowed to dry. This patch can then be placed on skin with the gauze side in direct contact with the skin for a period of time and can be held in place covered with a simple occlusive such as plastic wrap (e.g., Saran Wrap) and adhesive tape. Such a patch may have many compositions. The matrix holding the antigen may be cotton gauze, combinations of rayon-nylon or other synthetic materials and may have occlusive solid backings including polyvinyl chloride, rayons, other plastics, gels, creams, emulsions, waxes, oils, parafilm, rubbers (synthetic or natural), cloths, or membranes. The patch can be held onto the skin and components of the patch can be held together using various adhesives that are well known.

A liquid or quasi-liquid formulation may be applied directly to the skin and allowed to air dry; rubbed into the skin or scalp; placed on the ear, inguinal, or intertriginous regions, especially in animals; placed on the anal/rectal tissues; held in place with a dressing, patch, or absorbent material; immersion; otherwise held by a device such as a stocking, slipper, glove, or shirt; or sprayed onto the skin to maximize contact with the skin. But, in this invention, a formulation with at least one active ingredient in solid form is preferred. The formulation may be applied in an absorbent dressing or gauze. The formulation may be covered with an occlusive dressing such as, for example, AQUAPHOR (an emulsion of petrolatum, mineral oil, mineral wax, wool wax, panthenol, bisabol, and glycerin from Beiersdorf, Inc.), plastic film, COMFEEL (Coloplast) or vaseline; or a non-occlusive dressing such as, for example, TEGADERM (3M), DUODERM (3M) or OPSITE (Smith & Napheu). An occlusive dressing excludes the passage of water. The formulation may be applied to single or multiple sites, to single or multiple limbs, or to large surface areas of the skin by complete immersion. The formulation may be applied directly to the skin. Other substrates that may be used are pressure-sensitive adhesives such as acrylics, polyisobutylenes, and silicones. The formulation may be incorporated directly into such substrates, perhaps with the adhesive per se instead of adsorption to a porous pad (e.g., gauze) or bilious strip (e.g., filter paper).

A formulation with dry adjuvant may be produced in such a way that it is a universal adjuvant; that is, any vaccine may be added to the basic formulation with dry adjuvant. Such a universal adjuvant may be incorporated into a patch. The added vaccine may be aqueous and subsequently dried before immunization, or may be added in aqueous environment and applied to the vaccine.

Whether or not a patch is used, polymers added to the formulation may act as an excipient, stabilizer, and/or preservative of an active ingredient as well as reducing the concentration of the active ingredient that saturates a solution used to dissolve the dry form of the active ingredient. Such reduction occurs because the polymer reduces the effective volume of the solution by filling the "empty" space. Thus, quantities of antigen/adjuvant can be conserved without reducing the amount of saturated solution. An important thermodynamic consideration is that an active ingredient in the saturated solution will be "driven" into regions of lower concentration (e.g., through the skin). In solution, polymers can also stabilize and/or preserve the antigen/adjuvant-activity of solubilized ingredients of the formulation. Such polymers include ethylene or propylene glycol, vinyl pyrrolidone, and p-cyclodextrin polymers and copolymers.

### TRANSCUTANEOUS DELIVERY OF ANTIGEN

Efficient immunization can be achieved with the invention because transcutaneous delivery of antigen may target the Langerhans cell. These cells are found in abundance in the skin and are efficient antigen presenting cells leading to T-cell memory and potent immune responses. Because of the presence of large numbers of Langerhans cells in the skin, the efficiency of transcutaneous delivery may be related to the surface area exposed to antigen and adjuvant. In fact, the reason that transcutaneous immunization is so efficient may be that it targets a larger number of these efficient antigen presenting cells than intramuscular immunization.

We envision the invention will enhance access to immunization, while inducing a potent immune response. Because transcutaneous immunization does not require injection with a hypodermic needle (i.e., penetration to or through the dermis) and the complications and difficulties thereof, the requirements of trained personnel, sterile technique, and sterile equipment are reduced. Furthermore, the barriers to immunization at multiple sites or to multiple immunizations are diminished. Immunization by a single application of the formulation is also envisioned.

Immunization may be achieved using epicutaneous application of a simple formulation of antigen and adjuvant in solid form, optionally covered by an occlusive dressing, or by using other patch technologies. The immunization could be given by untrained personnel, and is amenable to self-application. Large-scale field immunization could occur given the easy accessibility to immunization. Additionally, a simple immunization procedure would improve access to immunization by pediatric patients and the elderly, and populations in Third World countries.

An object of the invention is to provide a novel means for immunization through intact skin without perforating the skin. Transcutaneous immunization according to the invention provides a method whereby antigens and adjuvant can be delivered to the immune system, especially specialized antigen presentation cells underlying the skin (e.g., Langerhans cells) without requiring a liquid-based delivery system.

For traditional vaccines, their formulations were injected through the skin with needles. Injection of vaccines using needles carries certain drawbacks including the need for sterile needles and syringes, trained medical personnel to administer the vaccine, discomfort from the injection, needle-bom diseases, and potential complications brought about by puncturing the skin with the potentially reusuable needles. Immunization through the skin without the use of hypodermic needles represents an advance for vaccine delivery by avoiding the hypodermic needles.

Moreover, transcutaneous immunization may be superior to immunization using hypodermic needles as more immune cells would be targeted by the use of several locations targeting large surface areas of skin. A therapeutically-effective amount of antigen sufficient to induce an immune response may be delivered transcutaneously either at a single cutaneous location, or over an area of skin covering multiple draining lymph node fields (e.g., cervical, axillary, inguinal, epitrochelear, popliteal, those of the abdomen and thorax). Such locations close to numerous different lymphatic nodes at locations all over the body will provide a more widespread stimulus to the immune system than when a small amount of antigen is injected at a single location by intradermal subcutaneous or intramuscular injection.

Antigen passing through or into the skin may encounter antigen presenting cells which process the antigen in a way that induces an immune response. Multiple immunization sites may recruit a greater number of antigen presenting cells and the larger population of antigen presenting cells that were recruited would result in greater induction of the immune response. It is conceivable that use of the skin may deliver antigen to phagocytic cells of the skin such as, for example, dermal dendritic cells, macrophages, and other skin antigen presenting cells; antigen may also be delivered to phagocytic cells of the liver, spleen, and bone marrow that are known to serve as the antigen presenting cells through the blood stream or lymphatic system.

Langerhans cells, dendritic cells, and macrophages may be specifically targeted using α2-macroglobulin bound antigen or other agents known to target APCs conjugated to or recombinantly produced as a protein fusion with adjuvant, antigen or both. Langerhans cells, dendritic cells, and macrophages may be specifically targeted using Fc receptor or the high affinity receptor for IgE (Bieber, 1997) conjugated to or recombinantly produced as a protein fusion with adjuvant; also, adjuvant may be conjugated to or recombinantly produced as a protein fusion with protein A or protein G (U.S. Pat Nos. 5,102,663, 5,141,742, and 5,262,177), prostate carcinoma (U.S. Pat No. 5,538,866), and lymphoma (U.S. Pat Nos. 4,816,249, 5,068,177, and 5,227,159). Vaccination with T-cell receptor oligopeptide may induce an immune response that halts progression of autoimmune disease (U.S. Pat Nos. 5,612,035 and 5,614,192; Antel et al., 1996; Vandenbark et al.,1996). U.S. Pat No. 5,552,300 also describes antigens suitable for treating autoimmune disease.

The following is meant to be illustrative of the invention; however, the practice of the invention is not limited or restricted in any way by the examples.

### EXAMPLES

### Immunization Procedure.

Mice of 6 to 8 weeks were shaved on the dorsum with a #40 clipper. This shaving could be done without any signs of trauma to the skin. The shaving-was done from the mid-thorax to just below the nape of the neck. The mice were then allowed to rest for 48 hours. Prior to this the mice had been ear-tagged for identification, and pre-bled to obtain a sample of pre-immune serum. Mice were also transcutaneously immunized without shaving by applying up to 50 µl of immunizing solution to each ear.

The mice were then immunized in the following way. Mice were anesthetized with 0.03-0.06 ml of a 20 mg/ml solution of xylazine and 0.5 ml of 100 mg/ml ketamine to prevent during immunization procedure. Mice were immobilized by this dose of anesthesia for approximately one hour. Larger doses or repetition may be used when immobilization for longer periods is needed. The mice were placed ventral side down on a warning blanket.

Immunizing solution was then placed on the dorsal shaved skin of a mouse in the following manner: a 1.2 cm x 1.6 cm stencil made of polystyrene was laid gently on the to target surface immunoglobulin of B cells. The result would be widespread distribution of antigen to antigen presenting cells to a degree that is rarely, if ever achieved, by current immunization practices.

Genetic immunization has been described in U.S. Pat. Nos. 5,589,466, 5,593,972, and 5,703,055. The nucleic acid(s) contained in the formulation may encode the antigen, the adjuvant, or both. The nucleic acid may or may not be capable of replication; it may be non-integrating and non-infectious. For example, the nucleic acid may encode a fusion polypeptide comprising antigen and a ubiquitin domain to direct the immune response to a class I restricted response. The nucleic acid may further comprise a regulatory region operably linked to the sequence encoding the antigen or adjuvant. The nucleic acid may be added with an adjuvant. The nucleic acid may be complexed with an agent that promotes transfection such as cationic lipid, calcium phosphate, DEAE-dextran, polybrene-DMSO, or a combination thereof; also, immune cells can be targeted by conjugation of DNA to Fc receptor or protein A/G, or attaching DNA to an agent linking it to α₂-macroglobulin or protein A/G or similar APC targeting material. The nucleic acid may comprise regions derived from viral genomes. Such materials and techniques are described by Kriegler (1990) and Murray (1991).

An immune response may comprise humoral (i.e., antigen-specific antibody) and/or cellular (i.e., antigen-specific lymphocytes such as B cells, CD4+ T cells, CD8+ T cells, CTL, Th1 cells, Th2 cells, and/or T_{DTH} cells) effector arms. Moreover, the immune response may comprise NK cells that mediate antibody-dependent cell-mediated cytotoxicity (ADCC).

The immune response induced by the formulation of the invention may include the elicitation of antigen-specific antibodies and/or cytotoxic lymphocytes (CTL, reviewed in Alving and Wassef, 1994). Antibody can be detected by immunoassay techniques, and the detection of various isotypes (e.g., IgM, IgD, IgA1, IgA2, secretory IgA, IgE, IgG1, IgG2, IgG3, or IgG4) may be expected. An immune response can also be detected by a neutralizing assay. Antibodies are protective proteins produced by B lymphocytes. They are highly specific, generally targeting one epitope of an antigen. Often, antibodies play a role in protection against disease by specifically reacting with antigens derived from the pathogens causing the disease. Immunization may induce antibodies specific for the immunizing antigen, such as cholera toxin.

CTLs are particular protective immune cells produced to protect against infection by a pathogen. They are also highly specific. Immunization may induce CTLs specific for the antigen, such as a synthetic oligopeptide based on a malaria protein, in association with self-major histocompatibility antigen. CTLs induced by immunization with the transcutaneous delivery system may kill pathogen infected cells. Immunization may also produce a memory response as indicated by boosting responses in antibodies and CTLs, lymphocyte proliferation by culture of lymphocytes stimulated with the antigen, and delayed type hypersensitivity responses to intradermal skin challenge of the antigen alone.

In a viral neutralization assay, serial dilutions of sera are added to host cells which are then observed for infection after challenge with infectious virus. Alternatively, serial dilutions of sera may be incubated with infectious titers of virus prior to innoculation of an animal, and the innoculated animals are then observed for signs of infection.

The transcutaneous immunization system of the invention may be evaluated using challenge models in either animals or humans, which evaluate the ability of immunization with the antigen to protect the subject from disease. Such protection would demonstrate an antigen-specific immune response. In lieu of challenge, achieving anti-diphtheria antibody titers of 5 IU/ml or greater is generally assumed to indicate optimum protection and serves as a surrogate marker for protection (Plotkin and Mortimer, 1994).

Furthermore, the Plasmodium falciparum challenge model may be used as to induce an antigen-specific immune response in humans. Human volunteers may be immunized using the transcutaneous immunization system containing oligopeptides or proteins (polypeptides) derived from the malaria parasite, and then exposed to malaria experimentally or in the natural setting. The Plasmodium yoelii mouse malaria challenge model may be used to evaluate protection in the mouse against malaria (Wang et al., 1995).

Cholera toxin-specific or LT specific IgG or IgA antibody may provide protection against cholera toxin challenge (Pierce, 1978; Pierce and Reynolds, 1974) and LT specific IgG or IgA is known to protect against ETEC related diarrheal disease.

Vaccination has also been used as a treatment for cancer, allergies, and autoimmune disease. For example, vaccination with a tumor antigen (e.g., prostate specific antigen) may induce an immune response in the form of antibodies, CTLs and lymphocyte proliferation which allows the body's immune system to recognize and kill tumor cells. Tumor antigens useful for vaccination have been described for melanoma back and a saline-wetted sterile gauze was used to partially wet the skin (this allowed even application of the immunizing solution), the immunizing solution was then applied with a pipet to the area circumscribed by the stencil to yield a 2 cm² patch of immunizing solution. Alternatively, immunizing solution is evenly applied the ear. Care was used not to scrape or rub the skin with the pipet tip. The immunizing solution was spread around the area to be covered with the smooth side of the pipet tip.

About 100 µl of immunizing solution was left on the back of the mouse for one hour. The mouse was then held gently by the nape of the neck and the tail under a copious stream of lukewarm tap water (about one liter) and washed. The mouse was then gently patted dry with a piece of sterile gauze and a second washing was performed; the mouse was then patted dry a second time and left in the cage. No adverse effects from the shaving, anesthesia, immunization, or washing procedures were observed. Neither erythema nor induration was seen at the immunization site for up to 72 hours after exposure to antigen. Immunization using the ear is performed as described above except that fur is not removed prior to immunization.

The production of dry formulations and patches of the invention, as well as their application, are described below.

### Antigen

The following antigens may be used for immunization or ELISA: cholera toxin or CT (List Biologicals, Campbell, CA, Cat #101B), CT B subunit (List Biologicals, Campbell, CA, Cat #BT01), CT A subunit (List Biologicals, Campbell, CA, Cat #102A), CT A subunit (Calbiochem, Cat #608562), pertussis toxin (List Biologicals, Campbell, CA), tetanus fragment C or tetC (List Biologicals, Campbell, CA), tetanus toxoid (List Biologicals, Campbell, CA), tetanus toxin (List Biologicals, Campbell, CA), *Pseudomonas* exotoxin A (List Biologicals, Campbell, CA), diphtheria toxoid (List Biologicals), *E. coli* heat-labile enterotoxin (Sigma, St. Louis, MO, lot #9640625), bovine serum albumin or BSA (Sigma, St. Louis, MO, Cat #3A-4503), and *Hemophilus influenza,* B conjugate (Connaught, lot#6J81401). They are mixed with sterile PBS or normal saline to dissolve (i.e., a liquid form), if the antigen is not intended to be provided in dry form.

### ELISA - IgG (H+L)

Antibodies specific for the described antigens were determined using ELISA in a technique similar to Glenn et al. (1995). All antigens were dissolved in sterile saline at a concentration of 2 µg/ml. Fifty microlilters of this solution (0.1 µg) per well was put on IMMULON-2 polystyrene plates (Dynatech, Chantilly, VA) and incubated at room temperature overnight. The plates were then blocked with a 0.5% casein/0.05% Tween 20 blocking buffer solution for one hour. Sera was diluted with 0.5% casein/0.05% Tween 20 diluent; dilution series were done in columns on the plate. Incubation was for two hours at room temperature.

The plates were then washed in a PBS-0.05% Tween 20 wash solution four times, and goat anti-mouse IgG (H+L) horseradish peroxidase (HRP)-linked (Bio-Rad, Richmond, CA, Cat #170-6516) secondary antibody was diluted in casein diluent at a dilution of 1/500 and left on the plates for one hour at room temperature. The plates were then washed four times in the PBS-Tween wash solution. One hundred microliters of 2,2'-azino-di-(3-ethyl-benzthiazolone) sulphonic acid substrate (ABTS, Kirkegaard and Perry, Gaithersburg, MD) were added to each well and the plates were read at 405 nm after about 30 minutes of development. Results are reported as the geometric mean of individual sera and standard error of the mean of ELISA units (the inverse serum dilution at which the absorbance in equal to 1.0) or as individual antibody responses in ELISA units. In all cases, the ELISA assays are conducted to discount the role of cross reactivity between coadministered antigens.

### ELISA - IgG(γ), IgM(µ), and IgA(α)

IgG(γ), IgM(µ) and IgA(α) anti-CT antibody levels are determined using ELISA with a technique similar to Glenn et al. (1995). CT is dissolved in sterile saline at a concentration of 2 µg/ml. Fifty microliters of this solution (0.1 µg) per well are put on IMMULON-2 polystyrene plates (Dynatech Laboratories, Chantilly, VA) and are incubated at room temperature overnight. The plates are then blocked with a 0.5% casein-Tween 20 blocking buffer solution for one hour. Sera is diluted in casein diluent, and serial dilutions are done on the plate. This is incubated for two hours at room temperature.

The plates are then washed in a PBS-Tween wash solution four times and goat anti-mouse IgG(γ) HRP-linked (Bio-Rad, Richmond, CA, Cat #172-1038), goat anti-mouse IgM(µ) HRP-linked (Bio-Rad, Richmond, CA, Cat #172-1030), or goat anti-mouse IgA (Zymed, South San Francisco, CA) secondary antibody is diluted in casein diluent at a dilution of 1/1000 and is left on the plates for one hour at room temperature. The plates are then washed four times in a PBS-Tween wash solution. One hundred microliters of 2,2'-azino-di-(3-ethyl benzthiazolone) sulphonic acid substrate from (ABTS, Kirkegaard and Perry, Gaithersburg, MD) are added to the wells and the plates are read at 405 nm. Results are reported as the geometric mean of individual sera and standard error of the mean of ELISA units (the inverse serum dilution at which the absorbance in equal to 1.0).

### ELISA - IgG Subclasses

Antigen-specific IgG (IgG, IgG2a, IgG2b, and IgG3) subclass antibody against CT, LT, ETA, and BSA is performed as described by Glenn et al. (1995). The solid phase ELISA is performed in IMMULON-2 polystyrene plates (Dynatech, Chantilly, VA). Wells are incubated with the respective antigens at 0.1 µg/50 µl in saline overnight, and then are blocked with 0.5% casein-Tween 20. Individual mouse sera diluted in 0.5% casein are serially diluted, and are incubated at room temperature for four hours. Secondary antibody consists of horseradish peroxidase-conjugated goat anti-mouse isotype-specific antibody (IgG1, IgG2a, IgG2b, IgG3, The Binding Site, San Diego, CA). A standard curve for each subclass is determined using mouse myeloma IgG1, IgG2a, IgG2b, and IgG3 (The Binding Site, San Diego, CA). Standard wells are coated with goat anti-mouse IgG (H+L) (Bio-Rad, Richmond, CA, Cat #172-1054) to capture the myeloma IgG subclass standards which are added in serial dilutions. The myeloma IgG subclass is also detected using the peroxidase-conjugated goat anti-mouse subclass-specific antibody. Both the test sera and myeloma standards are detected using 2,2'-azino-di-(3-ethyl-benzthiazolone) sulphonic acid (ABTS, Kirkegaard and Perry, Gaithersburg, MD) as substrate. Absorbances are read at 405 nm. Individual antigen specific subclasses are quantitated using the values from the linear titration curve computed against the myeloma standard curve and are then reported as µg/ml.

### ELISA - IgE

Antigen-specific IgE antibody quantitation is performed using a protocol from Pharmingen Technical Protocols, page 541 of the Research Products Catalog, 1996-1997 (Pharmingen, San Diego, CA). Fifty microliters of 2 µg/ml purified anti-mouse IgE capture mAb (Pharmingen, Cat #02111D) in 0.1 M NaHCO₃ (pH 8.2) are added to IMMUNO plates (Nunc, Cat #12-565-136). Plates are incubated overnight at room temperature, are washed three times with PBS-Tween 20, are blocked with 3% BSA in PBS for two hours, and are washed three times with PBS-Tween. Sera are diluted in 1% BSA in PBS, are added at dilutions of 1/100, and are diluted serially down the columns (e.g., 1/100, 1/200, et cetera). Purified mouse IgE standards (Pharmingen, Cat #0312D) are added with a starting dilution of 0.25 µg/ml and are serially diluted down the columns. Plates are incubated for two hours and are washed five times with PBS-Tween.

Biotinylated anti-mouse IgE mAB (Pharmingen, Cat #02122D) to 2 µg/ml in 1% BSA in PBS is incubated for 45 minutes and is washed five times with PBS-Tween. Avidin-peroxidase (Sigma A3151,1:400 of 1 mg/ml solution) is added for 30 min and plates are washed six times with PBS-Tween. Both the test sera and IgE standards are detected using 2,2'-azino-di-(3-ethyl-benzthiazolone) sulphonic acid (ABTS, Kirkegaard and Perry, Gaithersburg, MD) as substrate. Absorbances are read at 405 nm. Individual antigen specific subclasses are quantitated using the values from the linear titration curve computed against the IgE standard curve and are reported as µg/ml.

### Lung Washes and Stool Collection

Lung washes are obtained after sacrificing the mouse on the day of challenge. The trachea is transected, a 22 gauge polypropylene tube is inserted, and PBS is infused to gently inflate the lungs, The wash solution is withdrawn, then is reinfused for a total of three cycles, and then is stored at -20°C. Stool pellets are collected the day before challenge after spontaneous defecation. Pellets are weighed, are homogenized in 1 ml of PBS per 100 µg fecal material, are centrifuged, and supernatant is collected and then is stored at -20°C until assayed.

### Toxin Challenge

Mice are anesthetized with xylazine:ketamine and are challenged intranasally with 20 µl of CT (Calbiochem, La Jolla, CA) at 1 mg/ml in 10 mM TRIS (pH 7.5). Mice are challenged under anesthesia by intranasally administering 20 µg in buffer divided equally between each nare. Following challenge, mice are observed daily and both morbidity and mortality are recorded.

### Statistical Analysis

Unless otherwise indicated, data are represented as the geometric mean and SEM. Comparison between antibody titers in groups is performed using either paired or unpaired, one-tailed t tests and p values < 0.05 are regarded as significant. For challenge studies, the groups are compared by Fishers Exact test (SigmaStat, SPSS, Chicago, IL).

### Production of a Patch

Release liner (CO55, R&D material) was removed from acrylic adhesive laminate (K0021F, R&D material #S96I006), adhesive laminate was applied to backing (1012, R&D material), rolled, and the carrier paper strip was removed to expose the acrylic adhesive laminate. Four-ply gauze (Johnson & Johnson) in squares of 4 in. x 4 in. was rolled on the aforementioned backing held by acrylic adhesive laminate. One cm² round units were punched from the gauze (1) on backing (2) suitable for use with a single dose of formulation.

Release liner was removed from additional acrylic adhesive laminate, acrylic adhesive laminate was applied to TEGADERM occlusive dressing of CoTran 9701 (2 mil P.U.T., R&D material SLP#P261450143), rolled, and the carrier paper strip was removed to expose the acrylic adhesive laminate. A unit of gauze (1) on backing (2) was placed with tweezers on the aforementioned occlusive dressing (3) with the gauze-side up and the dressing-side down, held together by acrylic adhesive laminate. The gauze-side was covered with the release liner previously removed, rolled, and cut in rectangles of 2 cm x 2.5 cm with the unit in the center.

The patch constructed as described above is shown schematically in cross-section in Fig. 1. It can be prepared under aseptic conditions and stored at room temperature. The patch can be kept sterile by covering the patch with release liner (4) on the gauze-side and carrier paper (5) on the dressing-side.

### Example 1. Immunization of mice following epicutaneous application of lyophilized antigen, 160 µg of cholera toxin, to the skin.

C57BL6 mice were immunized using cholera toxin (CT) in the following manner: 2 mg of lyophilized CT (List Biological, Campbell, CA) was carefully removed from the original vial, weighed on a piece of paper (1.28 mg recovered) and divided into eight approximately equal parts of 160 µg each. Mice that were immunized with powder had 160 µg of CT carefully brushed off the paper onto the skin. The mice were anesthetized and shaved prior to immunization, and the immunizing powder was left on the skin for one hour, after which the mice were thoroughly washed. Pretreatment of the skin with water essentially involved wetting the skin for 5 minutes, blotting the skin dry, and placing the immunizing powder or solution on the skin. Mice immunized with liquid were immunized with 100 µl of 1 mg/ml CT in saline. Antibodies were detected by ELISA, as described, two weeks after the initial immunization. Only a single immunization was required. The geometric mean (geo mean) was calculated from the subtracted titers (14-day titer minus prebleed). See Table 1 for results.

The mice immunized with a saline solution of CT demonstrated typical immune responses as previously shown using transcutaneous immunization. When powder was placed on the skin, mice clearly developed high levels of antibodies. When the skin is hydrated prior to immunization, both the antigen delivered in aqueous form and antigen delivered in powder form were able to induce very high levels of antibodies. But mice immunized with the dry powder achieved consistently higher levels of antibodies.

**Table 1.**

| ear tag # | pretreatment | Antigen form | anti-CT IgG (ELISA units) | | |
|---|---|---|---|---|---|
| | | | prebleed | 14-day titer | geo mean |
| 967 | none | liquid | <5 | 2637 | 1465 |
| 969 | none | liquid | | 814 | |
| | | | | | |
| 996 | none | powder | <5 | 11395 | 4957 |
| 997 | none | powder | | 3223 | |
| 998 | none | powder | | 10067 | |
| 999 | none | powder | | 1633 | |
| | | | | | |
| 970 | H₂O | liquid | <5 | 12316 | 25356 |
| 971 | H₂O | liquid | | 26777 | |
| 965 | H₂O | liquid | | 49434 | |
| | | | | | |
| 961 | H₂O | powder | <5 | 26966 | 29490 |
| 962 | H₂O | powder | | 39211 | |
| 963 | H₂O | powder | | 26612 | |
| 964 | H₂O | powder | | 26879 | |

### Example 2. Immunization of mice following epicutaneous application of lyophilized antigen, 50 µg of cholera toxin, to the skin.

**Table 2.**

| Ear tag # | pretreatment | antigen form | anti-CT IgG (ELISA units) | | |
|---|---|---|---|---|---|
| | | | prebleed | 14-day titer | geo mean |
| 11707 | none | liquid | <10 | 2271 | 251 |
| 11708 | none | liquid | | 327 | |
| 11709 | none | liquid | | 247 | |
| 11710 | none | liquid | | 286 | |
| 11711 | none | liquid | | 19 | |
| | | | | | |
| 11712 | none | powder | <10 | 53 | 555 |
| 11713 | none | powder | | 1750 | |
| 11714 | none | powder | | 954 | |
| 11715 | none | powder | | 1731 | |
| 11716 | none | powder | | 342 | |
| | | | | | |
| 11717 | H₂O | liquid | <10 | 8645 | 11826 |
| 11718 | H₂O | liquid | | 14958 | |
| 11719 | H₂O | liquid | | 13622 | |
| 11720 | H₂O | liquid | | 13448 | |
| 11721 | H₂O | liquid | | 9765 | |
| | | | | | |
| 11722 | H₂O | powder | <10 | 4614 | 4487 |
| 11723 | H₂O | powder | | 7451 | |
| 11724 | H₂O | powder | | 2536 | |
| 11725 | H₂O | powder | | 3580 | |
| 11726 | H₂O | powder | | 5823 | |
| | | | | | |
| 11727 | alc/H₂O | liquid | <10 | 4656 | 7595 |
| 11728 | alc/H₂O | liquid | | 8131 | |
| 11729 | alc/H₂O | liquid | | 3728 | |
| 11730 | alc/H₂O | liquid | | 11335 | |
| 11731 | alc/H₂O | liquid | | 15797 | |
| | | | | | |
| 11732 | alc/H₂O | powder | <10 | 22100 | 7327 |
| 11733 | alc/H₂O | powder | | 6607 | |
| 11734 | alc/H₂O | powder | | 6204 | |
| 11735 | alc/H₂O | powder | | 7188 | |
| 11736 | alc/H₂O | powder | | 3244 | |

C57BL6 mice were immunized using cholera toxin (CT) in the following manner: 1 mg of lyophilized CT (List Biological, Campbell, CA) was carefully removed from the original vial, onto a piece of paper and divided into 20 equal parts of 50 µg each. Mice that were immunized with powder had 50 µg of CT carefully brushed off the paper onto the skin. The mice were anesthetized and shaved prior to immunization, and the immunizing powder was left on the skin for one hour, after which the mice were thoroughly washed. Pretreatment of the skin with water (H₂O) essentially involved wetting the skin for 5 minutes, blotting the skin dry with gauze, and placing the immunizing powder or solution on the skin. Mice that were swabbed with alcohol (alc) prior to immunization has an isopropyl alcohol (70%) swab rubbed gently 20 times across the skin. Mice immunized with liquid were immunized with 50 µl of 1 mg/ml CT in saline. Antibodies were detected by ELISA, as described, two weeks after the initial immunization. Only a single immunization was required. The geometric mean (geo mean) was calculated from the subtracted titers (14-day titer minus prebleed). See Table 2 for results.

The mice immunized with a saline solution of CT demonstrated typical immune responses as previously shown using transcutaneous immunization. Again, when powder was placed on the skin, mice clearly developed high levels of antibodies. When the skin was hydrated prior to immunization both the antigen delivered in aqueous form and antigen delivered in powder form were able to induce high levels of antibodies. Mice immunized using the dry powder achieved very high levels of antibodies with consistently high responses. Alcohol swabbing did not appear to interfere with the immune responses induced by powder immunization. This second experiment shows that dry formulations may be used in immunization, with or without pretreatment of the skin.

### Example 3. Immunization of mice following epicutaneous application of lyophilized antigen, 25 µg of cholera toxin, to the skin in an intermediate responder mouse strain.

BALB/c mice were immunized using cholera toxin (CT) in the following manner. 5 mg of lyophilized CT (List Biological, Campbell, CA) was dissolved in 1 ml of sterile water to make a 5 mg/ml solution. For the powder immunization, 5 µl of this solution was allowed to air dry at room temperature on a glass slide. The residual powder was then scraped off on the back of the mouse skin to be immunized. Thus, mice that were immunized with powder had 25 µg of CT carefully brushed off the slide onto the skin. Mice that were immunized with the dry patch had a 1 cm x 1 cm portion of a KIMWIPE tissue paper onto which 5 µl of 5 mg/ml CT was placed on a 4 cm x 4 cm square of plastic wrap (Saran) and allowed to air dry at room temperature. The tissue paper and plastic wrap were then placed with the tissue paper in direct contact with the skin of the mouse to be immunized and covered with the plastic wrap. The 'wet' patch used a tissue paper and plastic wrap 'patch' made by a similar technique, with the exception that 30 µl of sterile water was pipetted onto the dry patch after it was placed on the skin and plastic wrap was placed over the wet patch.

**Table 3.**

| ear tag # | pretreatment | antigen form | anti-CT IgG (ELISA units) | | |
|---|---|---|---|---|---|
| | | | prebleed | 14-day titer | geo mean |
| 806 | H₂O | liquid | <10 | 3177 | 1011 |
| 807 | H₂O | liquid | | 773 | |
| 809 | H₂O | liquid | | 266 | |
| 810 | H₂O | liquid | | 1976 | |
| 825 | H₂O | liquid | | 820 | |
| | | | | | |
| 821 | H₂O | dry patch | <10 | 37020 | 5315 |
| 822 | H₂O | dry patch | | 15090 | |
| 823 | H₂O | dry patch | | 12952 | |
| 824 | H₂O | dry patch | | 7419 | |
| 808 | H₂O | dry patch | | 79 | |
| | | | | | |
| 826 | none | wet patch | <10 | 11959 | 8042 |
| 827 | none | wet patch | | 24894 | |
| 828 | none | wet patch | | 11614 | |
| 829 | none | wet patch | | 10658 | |
| 830 | none | wet patch | | 913 | |
| | | | | | |
| 831 | H₂O | powder | <10 | 9504 | 4955 |
| 832 | H₂O | powder | | 4996 | |
| 833 | H₂O | powder | | 9841 | |
| 834 | H₂O | powder | | 358 | |
| 835 | H₂O | powder | | 17854 | |
| | | | | | |
| 836 | none | powder | <10 | 63 17 | |
| 837 | none | powder | | 6 | |
| 838 | none | powder | | 275 | |
| 839 | none | powder | | 3 | |
| 840 | none | powder | | 4 | |

The mice were anesthetized and shaved prior to immunization, and the immunizing powder was left on the skin for one hour, after which the mice were thoroughly washed. Pretreatment of the skin with water (H₂O) essentially involved wetting the skin for 5 minutes, blotting the skin dry with gauze, and placing the immunizing powder or solution on the skin. Mice immunized with liquid were immunized with 25 µl of 1 mg/ml CT in saline. Antibodies were detected by ELISA, as described, two weeks after the initial immunization. Only a single immunization was required. The geometric mean (geo mean) was calculated from the subtracted titers (14-day titer minus prebleed). See Table 3 for results.

The mice immunized with a saline solution of CT on pretreated skin demonstrated typical immune responses as previously shown using transcutaneous immunization with BALB/c mice (modest responder mice). Again, when powder was placed on the skin, mice clearly developed high levels of antibodies when the skin is hydrated prior to immunization. Both the patch placed on hydrated skin and the patch hydrated at the time of immunization achieved high levels of antibodies with consistently high responses. This third experiment shows that dry formulations may be used in patch form for immunization on the skin.

### Example 4. Serum anti-influenza A IgA- and IgG- titers in response to transcutaneous immunization with liquid or dried antigen formulations. C57BL/6 mice (five per group) were immunized with 50 µg of influenza strain A/Syd/5/97 (Flu A) and 60 µg of purified cholera toxin B subunit (pCTB) in either a liquid or solid formulation.

Briefly, backs of the mice from the distal aspect of the scapula to 0.5 cm above the base of the tail were shaved 48 hours prior to immunization. On the day of immunization, the mice were immobilized by injecting 30 µl of an anesthesia mixture yielding as final dose of about 83 mg/kg ketamine and 8.3mg/kg xylazine. The back of each mouse was hydrated by wiping 10 times with a water saturated gauze pad. A pool of water was left on the back for approximately 5 minutes. Excess water was blotted of with a dry gauze pad before applying antigen.

For dry patches, the antigen solution was mixed the day before immunization and a 1.25 x 2.5 cm clear adhesive patch was cut from a placebo patch provided by Elan Corporation. Two hundred microliters of antigen/adjuvant in saline was applied to the adhesive surface placing 100 µl of antigen and air drying for several hours, followed by placement of another 100 µl and air drying overnight.

For the liquid patch groups, 200 µl of saline containing (Flu A/pCTB) or 100 µl of saline containing pCTB was applied to the back. The animals were immunized as described above at 0, 3 and 6 weeks. Serum was collected prior to any antigen/adjuvant exposure (i.e., prebleed) and three weeks after the third immunization. Antigen specific antibody titers were assessed by a solid phase ELISA using influenza A antigen as the coating protein. Samples were diluted 1:100 in diluent.

See Table 4 for results on serum IgA titers. Following immunization, animals from both the solid and liquid formulation groups developed elevated anti-influenza A IgA titers as compared to the titers observed in the prebleed samples or from mice immunized with adjuvant (pCTB) alone. Three animals from the dried patch group exhibited increases of 2-fold or more over the prebleed while only one animal from the liquid antigen group displayed a 2-fold increase.

**Table 4.**

| | | | Serum anti-influenza A IgA (OD 405 nm) | | | | |
|---|---|---|---|---|---|---|---|
| Flu A (60 µg) | Dry | Average | | | Eartag# | | |
| pCTB (50 µg) | patch | prebleed | | | | | |
| | | | 13646 | 13647 | 13648 | 13649 | 13650 |
| | | 0.065 | 0.039 | 0.173 | 0.151 | 0.082 | 0.261 |
| | | | | | | | |
| Flu A (60 µg) | Liquid | Average | | | Eartag# | | |
| pCTB (50 µg) | | prebleed | | | | | |
| | | | 13666 | 13667 | 13668 | 13669 | 13670 |
| | | 0.076 | 0.065 | 0.073 | 0.640 | 0.076 | 0.105 |
| | | | | | | | |
| pCTB (50 µg) | Liquid | Average | | | Eartag# | | |
| | | prebleed | | | | | |
| | | | 13641 | 13642 | 13643 | 13644 | 13645 |
| | | 0.057 | < 0.020 | 0.010 | 0.031 | 0.032 | 0.078 |

See Table 5 for results on serum IgG titers. Following immunization, animals from both the solid and liquid formulation groups developed elevated anti-influenza A IgG titers as compared to the titers observed in the prebleed samples from mice immunized with adjuvant (pCTB) alone. Results are reported in ELISA units, the inverse dilution at which the OD 405 nm measures 1.0. As compared to the antigen alone group in which the maximum titer was 47, two of five mice in the dried antigen group and four of five mice in the liquid antigen group developed elevated antigen specific IgG titers.

**Table 5.**

| | | | Serum anti-influenza A IgG (ELISA units) | | | | |
|---|---|---|---|---|---|---|---|
| Flu A (60 µg) | Dry | Average | | | Eartag# | | |
| pCTB (50 µg) | patch | prebleed | | | | | |
| | | | 13646 | 13647 | 13648 | 13649 | 13650 |
| | | 5 | 31 | 12 | 32 | 547 | 2060 |
| | | | | | | | |
| Flu A (60 µg) | Liquid | Average | | | Eartag# | | |
| pCTB (50 µg) | | prebleed | | | | | |
| | | | 13666 | 13667 | 13668 | 13669 | 13670 |
| | | 20 | 489 | 30 | 870 | 97 | 9192 |
| | | | | | | | |
| pCTB (50 µg) | Liquid | Average | | | Eartag# | | |
| | | prebleed | | | | | |
| | | | 13641 | 13642 | 13643 | 13644 | 13645 |
| | | 8 | 44 | 13 | 17 | 47 | 17 |

### Example 5. Serum IgG antigen-specific responses after transcutaneous immunization with liquid or dried antigen formulations. C57BL/6 mice (five per group) were immunized on the skin with 25 µg of heat labile enterotoxin (LT) and 100 µg of diphtheria toxoid (DT) in dried or liquid formulations.

Briefly, the backs of the mice from the distal aspect of the scapula to 0.5 cm above the base of the tail were shaved 48 hours prior to immunization. On the day of immunization, the mice were immobilized by injecting 30 µl of an anesthesia mixture yielding a final dose of about 83 mg/kg ketamine and 8.3mg/kg xylazine. The back of each mouse was hydrated by wiping 10 times with a water saturated gauze pad. A pool of water was left on the back for approximately 5 minutes. Excess water was blotted of with a dry gauze pad before applying antigen.

For the patch groups, the antigen solutions were mixed 48-72 hrs before immunization. Patches were constructed as follows. For groups 1-5, a 1.25 cm by 2.5 cm clear adhesive patch was cut from a placebo patch provided by Elan Corporation. Group 1, a 0.6 cm by 1.5 cm piece of KIMWIPE tissue paper was cut and placed onto an adhesive patch; 25 µl of antigen solution was applied to the tissue paper surface and allowed to air dry overnight. Group 2, a 0.6 cm by 1.5 cm piece of tissue paper was cut and placed onto an adhesive patch; 25 µl of antigen solution was applied to the tissue paper surface and lyophilized overnight. Group 3, 25 µl of antigen solution was applied to the adhesive surface and allowed to air dry overnight. Group 4, 25 µl of antigen solution was applied to the adhesive surface and lyophilized overnight. Group 5, 1/5^{th} of a vial of DT was mixed with 1/40th of a vial of LT and placed directly onto the adhesive surface. Group 7, 1/5^{th} of a vial of DT was mixed with 1/40th of a vial of heat labile enterotoxin and placed directly onto the back of the mice. Group 9, 25 µl of saline containing the antigen was applied to the animal's back.

The mice were immunized as described above at 0, 3 and 5 weeks. Serum was collected prior to any antigen/adjuvant exposure (i.e., prebleed) and four weeks after the third immunization. Antigen-specific antibody titers were assessed by a solid phase ELISA using LT or DT antigen as the coating protein. Samples were serially diluted starting at 1:100 in diluent.

Results in Tables 6 and 7 are reported in ELISA units, the inverse dilution at which the OD 405 nm measures 1.0. Following immunization, animals from both the solid and liquid formulation groups developed elevated anti-LT (Table 6) and anti-DT (Table 7) IgG titers as compared to the average titer of prebleed samples (< 10 units). Together these results indicate that both dried and lyophilized antigen/adjuvant formulations can be used to deliver antigen through the skin and that the antigen can be applied in a dry, powder form, directly or on a simple adhesive backing.

**Table 6.**

| Group | Patch Materials | Antigen/Adjuvant formulation | Serum anti-LT IgG (ELISA units) | | | | |
|---|---|---|---|---|---|---|---|
| 1 | Adhesive backing | Dried on tissue paper | | | Eartag# | | |
| | | | 12601 | 12265 | 12266 | 12268 | 12269 |
| | | | 51839 | 18049 | 15498 | 19770 | 27196 |
| 2 | Adhesive backing | Lyophilized on tissue paper | | | Eartag# | | |
| | | | 12602 | 12271 | 12272 | 12273 | 12274 |
| | | | 9507 | 27188 | 4562 | 19975 | 11204 |
| 3 | Adhesive backing | Dried on adhesive backing | | | Eartag# | | |
| | | | 12603 | 12604 | 12276 | 12778 | 12279 |
| | | | 18305 | 7847 | 21263 | 20013 | 43500 |
| 4 | Adhesive backing | Lyophilized on adhesive backing | | | Eartag# | | |
| | | | 12605 | 12606 | 12281 | 12282 | 12283 |
| | | | 14554 | 14720 | 26337 | 34825 | 20509 |
| 5 | Adhesive backing | Powder on adhesive backing | | | Eartag# | | |
| | | | 12285 | 12286 | 12289 | 12619 | 12620 |
| | | | 35787 | 45075 | 16326 | 67213 | 48214 |
| 7 | None | Powder | | | Eartag# | | |
| | | | 12295 | 12298 | 400 | 622 | 623 |
| | | | 68768 | 27596 | 31289 | 40326 | 66530 |
| 9 | None | Liquid | | | Eartag# | | |
| | | | 12305 | 12308 | 12309 | 12607 | 12625 |
| | | | 17049 | 47980 | 75772 | 30734 | 13212 |

**Table 7.**

| Group | Patch Materials | Antigen/Adjuvant formulation | Serum anti-DT IgG (ELISA units) | | | | |
|---|---|---|---|---|---|---|---|
| 1 | Adhesive backing | Dried on tissue paper | | | Eartag# | | |
| | | | 12601 | 12265 | 12266 | 12268 | 12269 |
| | | | 16909 | 27135 | 43525 | 14166 | 21432 |
| 2 | Adhesive backing | Lyophilized on tissue paper | | | Eartag# | | |
| | | | 12602 | 12271 | 12272 | 12273 | 12274 |
| | | | 2697 | 662 | 4776 | 539 | 1394 |
| 3 | Adhesive backing | Dried on adhesive backing | | | Eartag# | | |
| | | | 12603 | 12604 | 12276 | 12778 | 12279 |
| | | | 58504 | 883 | 12063 | 27061 | 6544 |
| 4 | Adhesive backing | Lyophilized on adhesive backing | | | Eartag# | | |
| | | | 12605 | 12606 | 12281 | 12282 | 12283 |
| | | | 17867 | 20747 | 21065 | 15623 | 14323 |
| 5 | Adhesive backing | Powder on adhesive backing | | | Eartag# | | |
| | | | 12285 | 12286 | 12289 | 12619 | 12620 |
| | | | 713 | 9787 | 601 | 3329 | 5178 |
| 7 | none | Powder | | | Eartag# | | |
| | | | 12295 | 12298 | 400 | 622 | 623 |
| | | | 35579 | 18946 | 20351 | 17804 | 8957 |
| 9 | none | Liquid | | | Eartag# | | |
| | | | 12305 | 12308 | 12309 | 12607 | 12625 |
| | | | 5421 | 18166 | 14061 | 40605 | 47666 |

### Example 6. Serum IgG antigen-specific responses after transcutaneous immunization with varying doses of heat labile enterotoxin (LT), and a constant amount of diphtheria toxoid (DT). C57BL/6 mice (five per group) were immunized on the skin with dried or liquid formulations containing from 6 µg to 200 µg of LT and 100 µg of DT.

Briefly, the backs of the mice from the distal aspect of the scapula to 0.5 cm above the base of the tail were shaved 48 hours prior to immunization. On the day of immunization, the mice were immobilized by injecting 30 µl of an anesthesia mixture yielding a final dose of about 83 mg/kg ketamine and 8.3mg/kg xylazine. The back of each mouse was hydrated by wiping 10 times with a water saturated gauze pad. A pool of water was left on the back for approximately 5 minutes. Excess water was blotted of with a dry gauze pad before applying antigen.

Groups 1-6, the antigen solutions were mixed 48-72 hrs before immunization. Group 10, the antigen was prepared on the day of immunization. For the groups receiving patches, a 1.25 cm by 2.5 cm piece of cellophane was cut from commercially available material. One cm² pieces of KIMWIPE tissue paper were cut and placed onto the cellophane pieces. Twenty-five microliters of saline containing 100 µg of DT and LT (200 µg, group 1; 100 µg group 2; 50 µg group 3; 25 µg, group 4; 12.5 µg, group 5; 6.2 µg, group 6) was placed onto the tissue paper surface of the tissue paper/cellophane patches. The antigen solution was allowed to dry at room temperature overnight. For group 10, a 25 µl volume of antigen solution was placed directly onto the mouse skin. In all groups, the formulation was applied for 30 minutes at which time the remaining formulation was removed by rinsing the surface of the immunization site with copious amounts of water. Serum was collected prior to any antigen/adjuvant exposure (i.e., prebleed) and four weeks after the third immunization. Antigen specific antibody titers were assessed by a solid phase ELISA using LT or DT antigen as the coating protein. Samples were serially diluted starting at 1:100 in diluent.

Following immunization, animals from both the solid and liquid formulation groups developed elevated anti-LT (Table 8) and anti-DT (Table 9) IgG titers as compared to the titers observed in the prebleed samples. Results are reported in ELISA units, the inverse dilution at which the OD 405 nm measures 1.0. Together these results indicate that dried antigen/adjuvant formulations can be used to deliver antigen through the skin during epicutaneous immunization and that small amounts of adjuvant (LT) can elicit elevated antibody titers.

**Table 8.**

| Group | Dose of LT | Antigen Form | | Serum anti-LT IgG (ELISA units) | | | | |
|---|---|---|---|---|---|---|---|---|
| | | Dried on tissue | Average | | | Eartag# | | |
| 1 | 200 µg | paper | prebleed | 12350 | 12351 | 12352 | 12353 | 12354 |
| | | Cellophane backing | <10 | 62019 | 38733 | 71911 | 59745 | 128381 |
| | | Dried on tissue | Average | | | Eartag# | | |
| 2 | 100 µg | paper | prebleed | 12355 | 12356 | 12357 | 12358 | 12359 |
| | | Cellophane backing | <(0 | 53371 | 45852 | 37664 | 82222 | 21884 |
| | | Dried on tissue | Average | | | Eartag# | | |
| 3 | 50 µg | paper | prebleed | 12610 | 12611 | 12612 | 12363 | 12364 |
| | | Cellophane backing | <10 | 9852 | 30596 | 48966 | 27265 | 60935 |
| | | Dried on tissue | Average | | | Eartag# | | |
| 4 | 25 µg | paper | prebleed | 12613 | 12614 | 12366 | 12367 | 12368 |
| | | Cellophane backing | <10 | 12182 | 10279 | 3349 | 41821 | 42470 |
| | | Dried on tissue | Average | | | Eartag# | | |
| 5 | 12.5 µg | paper | prebleed | 12370 | 12371 | 12372 | 12373 | 12374 |
| | | Cellophane backing | <10 | 656 | 5433 | 213 | 5354 | 3835 |
| | | Dried on tissue | Average | | | Eartag# | | |
| 6 | 6.2 µg | paper | prebleed | 12375 | 12377 | 1239 | 12615 | 12616 |
| | | Cellophane backing | <10 | 659 | 34 | 1578 | 24 | 222 |
| | | | Average | | | Eartag# | | |
| 10 | 25 µg | Liquid | prebleed | 12395 | 12396 | 12397 | 12398 | 12399 |
| | | | <10 | 21679 | 18972 | 36749 | 24148 | 28259 |

**Table 9.**

| Group | Dose of LT | Antigen Form | | Serum anti-DT IgG (ELISA units) | | | | |
|---|---|---|---|---|---|---|---|---|
| | | Dried on tissue | Average | | | Eartag# | | |
| 1 | 200 µg | paper | prebleed | 12350 | 12351 | 12352 | 12353 | 12354 |
| | | Cellophane backing | <10 | 3176 | 9920 | 35802 | 41296 | 37318 |
| | | Dried on tissue | Average | | | Eartag# | | |
| 2 | 100 µg | paper | prebleed | 12355 | 12356 | 12357 | 12358 | 12359 |
| | | Cellophane backing | <10 | 56490 | 3668 | 47103 | 51026 | 1276 |
| | | Dried on tissue | Average | | | Eartag# | | |
| 3 | 50 µg | paper | prebleed | 12610 | 12611 | 12612 | 12363 | 12364 |
| | | Cellophane backing | <10 | 5526 | 8576 | 69200 | 28790 | 4269 |
| | | Dried on tissue | Average | | | Eartag# | | |
| 4 | 25 µg | paper | prebleed | 12613 | 12614 | 12366 | 12367 | 12368 |
| | | Cellophane backing | <10 | 26624 | 49297 | 23119 | 5544 | 10882 |
| | | Dried on tissue | Average | | | Eartag# | | |
| 5 | 12.5 µg | paper | prebleed | 12370 | 12371 | 12372 | 12373 | 12374 |
| | | Cellophane backing | <10 | 33467 | 15225 | 801 | 32369 | 2649 |
| | | Dried on tissue | Average | | | Eartag# | | |
| 6 | 6.2 µg | paper | prebleed | 12375 | 12377 | 1239 | 12615 | 12616 |
| | | Cellophane backing | <10 | 23269 | 2902 | 10924 | 18 | 7341 |
| | | | Average | | | Eartag# | | |
| 10 | 25 µg | Liquid | prebleed | 12395 | 12396 | 12397 | 12398 | 12399 |
| | | | <10 | 56346 | 26444 | 72928 | 65058 | 10800 |

### Example 7. Serum IgG antigen-specific responses after transcutaneous immunization with constant amounts heat labile enterotoxin (LT) and diphtheria toxoid (DT) on patches of decreasing size. C57BL/6 mice (five per group) were immunized on the skin with 25 µg of LT and 100 µg of DT in dried or liquid formulations.

Briefly, the backs of the mice from the distal aspect of the scapula to 0.5 cm above the base of the tail were shaved 48 hours prior to immunization. On the day of immunization, the mice were immobilized by injecting 30 µl of an anesthesia mixture yielding a final dose of about 83 mg/kg ketamine and 8.3mg/kg xylazine. The back of each mouse was hydrated by wiping 10 times with a water saturated gauze pad. A pool of water was left on the back for approximately 5 minutes. Excess water was blotted off with a dry gauze pad before applying antigen. Groups 1-5, the antigen solutions were mixed 48-72 hrs before immunization. Group 6, the antigen was prepared on the day of immunization.

For the groups receiving patches, a 1.25 cm by 2.5 cm piece of cellophane was cut from commercially available material. Pieces of KIMWIPE tissue paper were cut into a series of smaller surface areas to allow for more concentrated delivery of the formulation. The dimensions of the tissue paper pieces were as follows: 1.00 cm² (group 1), 0.5 cm² (group 2), 0.25 cm² (group 3), 0.12 cm² (group 4), and 0.06 cm² (group 5). The cut pieces were then placed onto the cellophane backing. All groups received a constant dose of active ingredients, 25 µg LT and 100 µg DT, which was placed onto the tissue paper/cellophane patch in increasing concentrations to allow for the decreasing patch surface area. The volumes per patch per mouse were as follows: 50 µl (group 1), 25 µl (group 2), 12.5 µl (group 3), 6.3 µl (group 4), and 3.1 µl (group 5). The antigen solution was allowed to dry at room temperature overnight. For group 6, a 50 µl volume of solution was placed directly onto the mouse skin. In all groups, the formulation was applied for 30 minutes at which time the remaining formulation was removed by rinsing the surface of the immunization site with copious amounts of water.

Serum was collected prior to any antigen/adjuvant exposure (i.e., prebleed) and four weeks after the third immunization. Antigen specific antibody titers were assessed by a solid phase ELISA using LT or DT antigen as the coating protein. Samples were serially diluted starting at 1:100 in diluent.

Following immunization, animals from both the solid and liquid formulation groups developed elevated anti-LT (Table 10) and anti-DT (Table 11) IgG titers as compared to the titers observed in the prebleed samples. Results are reported in ELISA units, the inverse dilution at which the OD 405 nm measures 1.0. Together these results indicate that both dried and lyophilized antigen/adjuvant formulations can be used to deliver antigen through the skin during epicutaneous immunization and that the dried antigen can be applied on small surface areas and still yield significant immune responses.

**Table 10.**

| Group | Patch Surface Area | Antigen Form | | Serum anti-LT IgG (ELISA units) | | | | |
|---|---|---|---|---|---|---|---|---|
| | | Dried on tissue | Average | | | Eartag# | | |
| 1 | 1.00cm² | paper | prebleed | 12310 | 12311 | 12312 | 12313 | 12314 |
| | | Cellophane backing | <10 | 276 | 4252 | 14914 | 4076 | 18496 |
| | | Dried on tissue | Average | | | Eartag# | | |
| 2 | 0.50 cm² | paper | prebleed | 12315 | 12316 | 12317 | 12318 | 12319 |
| | | Cellophane backing | <10 | 6980 | 13057 | 678 | 5266 | 10352 |
| | | Dried on tissue | Average | | | Eartag# | | |
| 3 | 0.25 cm² | paper | prebleed | 12321 | 12322 | 12323 | 12609 | |
| | | Cellophane backing | <10 | 39141 | 16677 | 2214 | 9680 | |
| | | Dried on tissue | Average | | | Eartag# | | |
| 4 | 0.13 cm² | paper | prebleed | 12324 | 12325 | 12326 | 12327 | 12329 |
| | | Cellophane backing | <10 | 6200 | 2932 | 3288 | 13112 | 2859 |
| | | Dried on tissue | Average | | | Eartag# | | |
| 5 | 0.06 cm² | paper | prebleed | 12330 | 12331 | 12332 | 12333 | 12334 |
| | | Cellophane backing | <10 | 1508 | 1311 | 2072 | 1499 | 2573 |
| | | | Average | | | Eartag# | | |
| 6 | 1.00 cm² | Liquid | prebleed | 12335 | 12336 | 12337 | 12338 | 12339 |
| | | | <10 | 14984 | 7403 | 3663 | 42290 | 56247 |

**Table 11.**

| Group | Patch Surface Area | Antigen Form | | Serum anti-DT IgG (ELISA units) | | | | |
|---|---|---|---|---|---|---|---|---|
| | | Dried on tissue | Average | | | Eartag# | | |
| 1 | 1.00 cm² | paper | prebleed | 12310 | 12311 | 12312 | 12313 | 12314 |
| | | Cellophane backing | <10 | 3389 | 2259 | 3638 | 5551 | 7747 |
| | | Dried on tissue | Average | | | Eartag# | | |
| 2 | 0.50 cm² | paper | prebleed | 12315 | 12316 | 12317 | 12318 | 12319 |
| | | Cellophane backing | <10 | 71236 | 68949 | 6169 | 18233 | 67872 |
| | | Dried on tissue | Average | | | Eartag# | | |
| | 0.25 cm² | paper | prebleed | 12321 | 12322 | 12323 | 12609 | |
| | | Cellophane backing | <10 | 11771 | 33543 | 12415 | 12648 | |
| | | Dried on tissue | Average | | | Eartag# | | |
| 4 | 0.13 cm² | paper | prebleed | 12324 | 12325 | 12326 | 12327 | 12329 |
| | | Cellophane backing | <10 | 75433 | 155 | 8997 | 28730 | 7667 |
| | | Dried on tissue | Average | | | Eartag# | | |
| 5 | 0.06 cm² | paper | prebleed | 12330 | 12331 | 12332 | 12333 | 12334 |
| | | Cellophane backing | <10 | 4814 | 2122 | 471 | 13511 | 89 |
| | | | Average | | | Eartag# | | |
| 6 | 1.00 cm² | Liquid | prebleed | 12335 | 12336 | 12337 | 12338 | 12339 |
| | | | <10 | 38885 | 22697 | 24334 | 45101 | 34333 |

The foregoing examples demonstrate the advantage of using a dry formulation and various physical forms thereof in transcutaneous immunization. It should be noted, however, that the processes involved in achieving transcutaneous immunization as shown in our publications (Glenn et al., 1998ab; 1999); U.S. Pat. Nos. 5,910,306 and 5,980,898; WO 98/20734 and WO 99/43350; and U.S. Appln. Nos. 09/257,188; 09/309,881; 09/311,720; 09/316,069; and 09/337,746 might be adapted in the context of the invention.

### REFERENCES

Alving and Wassef (1994) AIDS Res. Hum. Retro., 10 (suppl. 2):S91-S94. Antel et al. (1996) Nature Medicine, 2:1074-1075.
Ausubel et aL (1996) Current Protocols in Molecular Biology. Wiley, New York. Ball et al. (1998) J. Virol., 72:1345-1353.
Bathurst et al. (1993) Vaccine, 11:449-456.
Bieber (1997) Intl. Arch. Allergy Immuonol.,113:30-34.
Black et aL (1979) Report and immunogenicity of Wellcome cholera toxoids in Bangladeshi volunteers, Bangladesh, Scientific Report No. 29, Dacca.
Blurn (1995) Digestion, 56:85-95.
Bodanszky (1993) Peptide Chemistry. Springer-Verlag, New York:
Bos (1997) Clin. Exp. Immunol., 107 (suppl. 1):3-5.
Burnette et al. (1994) In: Bioprocess Technology. (Burnette et al.), pp. 185-203.
Celluzzi and Falo (1997) J. Invest. Dermatol., 108:716-720.
Chang et al. (1989) Proc. Natl. Acad. Sci. USA, 86:6343-6347.
Chang et al. (1992) J. Immunol., 139:548-555.
Chang et al. (1994) J. Immunol., 152:3483-3490.
Chen et al. (1998) Infect. Immun., 66:1648-1653.
Clements and Finkelstein (1979) Infect. Immunol., 24:760-769.
Craig (1965) In: Proceedings of the Cholera Research Symposium, Honolulu, US Public Health Service Publication No. 1328, pp. 153-158.
Craig (1966) J. Bact., 92: 793-795.
Curlin et al. (1975) In: Proceedings of the 11th Joint Conference on Cholera. U.S. Japan Cooperative Medical Science Program.
Dahl (1996) In: Clinical Immunodermatology, 3rd Ed. Mosby, St. Louis, pp. 345-352. Delenda et al. (1994) J. Gen. Virol., 75:1569-1578.
Deprez et al. (1996) Vaccine, 14:375-382.
Deutscher (1990) Guide to Protein Purification. Academic Press, San Diego. Dickenson and Clements (1995) Infect. Immun., 63:1617-1623.
Douce et al. (1997) Infect. Immun., 65:28221-282218.
Dragunsky et al. (1992) Vaccine, 10:735-736.
Elson and Dertzbaugh (1994) In: Handbook of Mucosal Immunology. Academic Press, San Diego, p. 391.
Finkelstein and LoSpallutto (1969) J. Exp. Med., 130:185-202.
Fonseca et al. (1994) Vaccine, 12:279-285.
Frankenburg et al. (1996) Vaccine, 14:923-929.
Fries et al. (1992a) Proc. Natl. Acad. Sci. USA, 89:358-362.
Fries et al. (1992b) Infect. Immun., 60:1834-1839.
Fujita and Finkelstein (1972) J Infect Dis, 125:647-655.
Glenn et al. (1995) Immunol. Lett., 47:73-78.
Glenn et al. (1998a) Nature, 393:851.
Glenn et al. (1998b) J. Immunol., 161:3211-3214, 1998
Glenn et al. (1999) Infect. Immun., 67:1100-1106.
Goeddel (1990) Gene Expression Technology. Academic Press, San Diego. Gregoriadis (1993) Liposome Preparation and Related Techniques, 2nd Ed. CRC Press, Boca Raton.
Hanson and Roun (1992) In: Stability of Protein Pharmaceuticals, Vol. 3, Plenum Press, New York, pp. 209-233.
Herrington et al. (1991) Am. J. Trop. Med. Hyg., 45:695-701.
Herz et al. (1998) Intl. Arch. Allergy Immunol., 115:179-190.
Holmgren et al. (1975) Infect. Immun., 12: 463-470.
Howell and Miller (1983) J. Clin. Microbiol., 18:658-662.
Jahrling et al. (1996) Arch. Virol. Suppl., 11:135-140.
Janeway and Travers (1996). Immunobiology. Churchill Livingstone, New York.
Janson and Ryden (1997) Protein Purification, 2nd Ed. Wiley, New York. Jackson et al. (1993) Infec. Immun., 61:4272-4279.
John et al. (1996) Dev. Biol. Stand., 87:19-25.
Katkov (1996) Med. Clin. North Am., 80:189-200.
Khusmith et al. (1991) Science, 252:715-718.
Kleinau et al. (1994) Clin. Exp. Immunol., 96:281-284.
Kounnas et al. (1992) J. Biol. Chem., 267:12420-12423.
Kreig et al. (1995) Nature, 374:546.
Kriegler (1990) Gene Transfer and Expression. Stockton Press, New York. Kripke et al. (1987) J. Immunol., 145:2833-2838.
Krueger and Barbieri (1995) Clin. Microbiol. Rev., 8:34-47.
Lee and Chen (1994) Infect. Immun., 62:3594-3597.
Leung (1995) J. Invest. Dermatol., 105 (Suppl. 1):37S-42S.
Leung (1997) Clin. Exp. Immunol., 107 (Suppl. 1):25-30.
Lange et al. (1979) Infect Immun., 23:743-750.
Lieberman and Greenberg (1996) Adv. Pediatr. Infect. Dis., 11:333-363.
Lu et al. (1997) Vaccine Res., 6:1-13.
Malik et al. (1991) Proc. Natl. Acad. Sci. USA, 88:3300-3304.
Mast and Krawczynski (1996) Annu. Rev. Med., 47:257-266.
Mekalanos et al. (1979) J. Biol. Chem., 254:5855-5861.
Medzhitov and Janeway (1997) Curr. Opin. Immunol., 9:4-9.
Merson et al. (1980) Lancet, i:9312-932.
Migliorini et al. (1993) Eur. J. Immunol., 23:582-585.
Morein and Simons (1985) Vaccine, 3:83-93.
Moschella (1996) Cutaneous Medicine and Surgery. W.B. Saunders, Philadelphia. Moschella and Hurley (1992) Dermatology, 3rd Ed. Harcourt Brace Janovitch, Philadelphia.
Munoz et al. (1990) J. Exp. Med., 172:95-103.
Murphy et al. (1998) Cutan. Pathol., 25:30-34.
Murray (1991) Gene Transfer and Expression Protocols. Humana Press, Clifton, NJ. Nashar et al. (1997) Immunology, 91:572-578.
Nohria and Rubin (1994) Biotherapy, 7:261-269.
Northrup and Chisari (1972) J. Infect. Dis., 125:471-479.
Paul and Cevc (1995) Vaccine Res., 3:145-164.
Paul et al. (1995) Eur. J. Immunol., 25:3521-3524.
Pessi et al. (1991) Eur. J. Immunol., 24:2273-2276.
Peterson (1979) Infect. Immun., 26:594-598.
Pierce and Reynolds (1974) J. Immunol., 113:1017-11023.
Pierce and Reynolds (1978) J. Exp. Med., 148:195-206.
Pierce et al. (1976) Infect. Immun., 13:735-740.
Pierce et al. (1977) J. Infect. Dis., 135:888-896.
Pierce et al. (1978) Infect. Immun., 21:185-193.
Pierce et al. (1980) Infect. Immun., 27:632-637.
Pierce et al. (1983) Infect. Immun., 37I: 687-694.
Plotkin and Mortimer (1994) Vaccines, 2nd Ed. W.B. Saunders, Philadelphia. Ramiya et al. (1996) J. Autoimmun., 9:349-356.
Rappaport et al. (1976) Infect. Immun., 14:687-693.
Rappuoli et al. (1995) Intl. Archiv. Allergy Immunol., 108:327-333.
Rappuoli et al. (1996) Adv. Exp. Med. Biol., 397:55-60.
Ribi et al. (1988) Science, 239:1272-1276.
Richards et al. (1995) In: Vaccine Design, Plenum, New York.
Rietschel et al. (1994) FASEB J., 8:217-225.
Roberts and Walker (1993) In: Pharmaceutical Skin Penetration Enhancement. Marcel Dekker, New York.
Saloga et al. (1996a) J. Invest. Dermatol., 106:982-988.
Saloga et al. (1996b) Exp. Dermatol., 5:65-71.
Sanchez et al. (1999) Intl. J. Pharm., 185:255-266.
Sasaki et al. (1998) Clin. Exp. Immunol., 111:30-35.
Saukkonen et al. (1992) Proc. Natl. Acad. Sci. USA, 89:118-122.
Schneerson et al. (1996) Lancet, 348:1289-1292.
Scopes (1993) Protein Purification. Springer-Verlag, New York.
Seder and Paul (1994) Annu. Rev. Immunol., 12:635-673.
Shafara et al. (1995) Ann. Intern. Med., 125:658-668.
Skeiky et al. (1995) J. Exp. Med., 181:1527-1537.
Smedile et al. (1994) Prog. Liver Dis., 12:157-175.
Smucny et al. (1995) Am. J. Trop. Med. Hyg., 53:432-437.
Sniderman (1995) Crit. Rev. Immunol., 15:317-348.
Spangler (1992) Microbiol. Rev., 56:622-647.
Stacey et al. (1996) J. Immunol., 157:2116-2122.
Stoute et al. (1997) New Engl. J. Med., 336:86-91.
Summers and Smith (1987) A manual of methods for baculovirus vectors and insect cell culture procedure. Texas Agricultural Experiment Station Bulletin, No. 1555.
Svennerholm et al. (1978) Infect. Immun., 211-6.
Svennerholm et al. (1984) Bull. WHO, 62:909-918.
Svennerholm et al. (1982) Lancet, i:305-308.
Tam (1988) Proc. Natl. Acad. Sci. USA, 85:5409-5413.
Trach et al. (1997) Lancet, 349:231-235.
Udey (1997) Clin. Exp. Immunol., 107 (Suppl. 1):6-8.
Vajdy et al. (1995) J. Exp. Med., 181:41-53.
van Heyningen and Seal (1983) Cholera: The American Scientific Experience, 1947-1980. Westview Press, Boulder, Colorado.
Vandenbark et al. (1996) Nature Medicine, 2:1109-1115.
Vosika et al. (1984) Cancer Immunol. Immunother., 18:107-112.
Vreden et al. (1991) Am. J. Trop. Med. Hyg., 45:533-538.
Walters and Hadgraft (1993) Pharmaceutical Skin Penetration Enhancement, Marcel Dekker, New York.
Wang et al. (1995) J. Immunol., 154:2784-2793.
Wang et al. (1996) J. Immunol., 156:4079-4082.
White et al. (1993) Vaccine, 11:1341-1346.
Wiedermann et al. (1998) Clin. Exp. Immunol., 111:144-151.
Wiesmueller et al. (1991) Immunology, 72:109-113.
Wisdom (1994) Peptide Antigens. IRL Press, Oxford.
Zepter et al. (1997) J. Immunol., 159:6203-6208.
Zhang et al. (1995) Infect. Immun., 63:1349-1355.

## Claims

1. Formulation for transcutaneous immunisation comprising an antigen in dry form and an adjuvant in dry form, and whereby application of the formulation to intact skin, without perforation thereof, induces an immune response specific for the antigen, wherein said adjuvant comprises a bacterial ADP-ribosylating exotoxin or a ganglioside GM1-binding subunit thereof.

2. Formulation according to claim 1, wherein said antigen has a molecular weight greater than 500 daltons.

3. Formulation according to claim 1, wherein said antigen is derived from a pathogen selected from the group consisting of bacterium, virus, fungus and parasite.

4. Formulation according to claim 3, wherein the antigen is selected from the group consisting of: Bacillus anthracis, Camplyobacter, Vibrio cholerae, Clostridia including clostridium difficile, diphtheria, enterohemorrhagic E. coli, enterotoxigenic E. coli, Giardia, gonococcus, Helicobacter pylori or urease produced by H. pylori, Hemophilus influenza B, Hemophilus influenza non-typable, Legionella, meningococcus, Mycobacterium including those organisms responsible for tuberculosis, pertussis, pneumococcus, Salmonella, Shigella, Staphylococcus and their enterotoxins, Group A beta-hemolytic streptococcus, Streptococcus B, tetanus, Borrelia burgdorfi and Yersinia.

5. Formulation according to claim 3, wherein the antigen is selected from the group consisting of: adenovirus, dengue serotypes 1 to 4, ebola, enterovirus, hanta virus, hepatitis serotypes A to E, herpes simplex virus 1 or 2, human immunodeficiency virus, human papilloma virus, influenza, measles, Norwalk, Japanese equine encephalitis, papilloma virus, parvovirus B 19, polio, rabies, respiratory syncytial virus, rotavirus, rubella, rubeola, St. Louis encephalitis, vaccinia, viral expression vectors containing genes coding for other antigens such as malaria antigens, varicella, and yellow fever.

6. Formulation according to claim 1, wherein said antigen is a tumor antigen or an autoantigen.

7. Formulation according to claim 1, where said antigen is selected from the group consisting of carbohydrate, glycolipid, glycoprotein, lipid, lipoprotein, phospholipid, and polypeptide.

8. Formulation according to claim 1, wherein the formulation is comprised of an attenuated live virus and at least one antigen is expressed by the attenuated live virus.

9. Formulation according to claim 1, wherein said antigen is a polypeptide of greater than 500 daltons molecular weight.

10. Formulation according to claim 1, wherein said antigen is multivalent.

11. Formulation according to claim 1, wherein a single molecule is both an adjuvant and an antigen of the formulation.

12. Formulation according to any one of claims 1-7 or 9-11, wherein the formulation does not include a penetration enhancer, viral particle, liposome, or charged lipid.

13. Formulation according to any one of claims 1-11, wherein the formulation does include a chemical penetration enhancer that increases effectiveness of immunisation in comparison to use for a formulation lacking the chemical penetration enhancer.

14. Formulation according to any one of claims 1-13, whereby exposure of the intact skin to the adjuvant activates an underlying Langerhans cell.

15. Formulation according to any one of claims 1-14, whereby exposure of the intact skin to the adjuvant causes an underlying Langerhans cell to increase major histocompatibility complex class II expression.

16. Formulation according to any one of claims 1-14, whereby exposure of the intact skin to the adjuvant causes migration of an underlying Langerhans cell to a lymph node.

17. Formulation according to any one of claims 1-14, whereby exposure of the intact skin to the adjuvant signals an underlying Langerhans cell to mature into an antigen presenting cell.

18. Formulation according to any one of claims 1-14, whereby exposure of the intact skin to the adjuvant enhances antigen presentation to lymphocytes.

19. Formulation according to any one of the preceding claims further comprising an excipient, stabiliser, desiccant or preservative.

20. Formulation according to claim 19, comprising a preservative or stabiliser selected from the group consisting of sucrose, mannitol, sorbitol, trehalose, dextran and glycerin.

21. Formulation according to any one of claims 1-20 further comprising a dressing and thereby providing a patch form of the formulation.

22. Formulation according to claim 21, wherein the dressing is an occlusive dressing.

23. Formulation according to claim 21, wherein the intact skin covered by the dressing has a surface area which is larger than at least one draining lymph node field.

24. The dry formulation in accordance with any one of the preceding claims, for transcutaneous immunisation by applying the formulation to the intact skin of a subject without perforation thereof, and comprising enhancing penetration of at least one antigen or adjuvant through the skin with at least one physical or chemical penetration enhancer applied to the skin before, after and/or during application of the formulation to the skin.

25. The dry formulation of claim 24, comprising applying alcohol to, or hydrating, the intact skin prior to application of the formulation.

26. A method of producing a formulation in accordance with any one of claims 1-23 for transcutaneous immunisation comprising:
(a) providing at least one antigen and at least one adjuvant;
(b) dissolving at least the antigen and adjuvant to produce an immunologically active liquid;
(c) drying the immunologically-active liquid on a solid substrate to produce the formulation;
(d) wherein at least the antigen and adjuvant are in dry form at least until the formulation is applied to skin of a subject to be immunised, and wherein the adjuvant comprises a bacterial ADP-ribosylating exotoxin or a ganglioside GM1-binding subunit thereof.

27. A method according to claim 26, further comprising mixing all active ingredients together to produce a homogenous formulation.

28. A method according to claim 26 or 27, wherein the formulation is comprised essentially of one immunologically-active ingredient.

29. A method according to any one of claims 26-28, wherein the solid substrate is a dressing and the formulation is thereby provided as a patch.

30. A method according to any one of claims 26-29, wherein the formulation is produced under aseptic conditions suitable for production of a vaccine.

31. A method according to any one of claims 26-30, wherein a single application of the formulation is sufficient to induce a detectable antigen-specific immune response.

32. A method according to any one of claims 26-31, wherein the formulation is further comprised of at least one excipient, stabiliser, desiccant or preservative.

## Patentansprüche

1. Formulierung zur transkutanen Immunisierung, umfassend ein Antigen in trockener Form und einen Hilfsstoff in trockener Form, wobei das Auftragen der Formulierung auf intakte Haut ohne Perforation davon eine für das Antigen spezifische Immunantwort induziert, wobei der Hilfsstoff ein bakterielles ADP-ribosylierendes Exotoxin oder eine Gangliosid-GM1-bindende Untereinheit davon umfasst.

2. Formulierung gemäß Anspruch 1, wobei das Antigen ein Molekulargewicht von über 500 Dalton aufweist.

3. Formulierung gemäß Anspruch 1, wobei das Antigen aus einem Pathogen, ausgewählt aus der Gruppe, bestehend aus Bakterium, Virus, Fungus und Parasit, abgeleitet ist.

4. Formulierung gemäß Anspruch 3, wobei das Antigen ausgewählt ist aus der Gruppe, bestehend aus: Bacillus anthracis, Camplyobacter, Vibrio cholerae, Clostridia, einschließlich Clostridium difficile, Diphteria, enterohämorrhagisches E. coli, enterotoxigenes E. coli, Giardia, Gonococcus, Heliobacter pylori oder Urease, die von H. pylori hergestellt ist, Hemophilus influenza B, nichttypisierbares Hemophilus influenza, Legionella, Meningococcus, Mycobacterium, einschließlich jene Organismen, die für Tuberkulose verantwortlich sind, Pertussis, Pneumococcus, Salmonella, Shigella, Staphylococcus und deren Enterotoxine, beta-hämolytisches Streptococcus Gruppe A, Streptococcus B, Tetanus, Borrelia burgdorfi und Yersinia.

5. Formulierung gemäß Anspruch 3, wobei das Antigen ausgewählt ist aus der Gruppe, bestehend aus: Adenovirus, Dengue-Serotypen 1 bis 4, Ebola, Enterovirus, Hanta-Virus, Hepatitis Serotypen A bis E, Herpes-simplex-Virus 1 oder 2, humanes Immundefizienzvirus, humanes Papillomvirus, Influenza, Masern, Norwalk, japanische Pferde-Enzephalitis, Papillomvirus, Parovirus B19, Polio, Tollwut, Respiratory-Syncytial-Virus, Rotavirus, Röteln, Rubeola, St. Louis-Enzephalitis, Vaccinia, virale Expressionsvektoren, die Gene enthalten, die andere Antigene codieren, wie z. B. Malaria-Antigene, Windpocken und Gelbfieber.

6. Formulierung gemäß Anspruch 1, wobei das Antigen ein Tumorantigen oder ein Autoantigen ist.

7. Formulierung gemäß Anspruch 1, wobei das Antigen ausgewählt ist aus der Gruppe, bestehend aus Kohlenhydrat, Glycolipid, Glycoprotein, Lipid, Lipoprotein, Phospholipid und Polypeptid.

8. Formulierung gemäß Anspruch 1, wobei die Formulierung ein attenuiertes lebendes Virus und wenigstens ein Antigen, das von dem attenuierten lebenden Virus exprimiert wird, umfasst.

9. Formulierung gemäß Anspruch 1, wobei das Antigen ein Polypeptid mit einem Molekulargewicht von über 500 Dalton ist.

10. Formulierung gemäß Anspruch 1, wobei das Antigen multivalent ist.

11. Formulierung gemäß Anspruch 1, wobei ein einzelnes Molekül sowohl ein Hilfsstoff als auch ein Antigen der Formulierung ist.

12. Formulierung gemäß einem der Ansprüche 1-7 oder 9-11, wobei die Formulierung kein/keinen Penetrationsverstärker, virales Partikel, Liposom oder geladenes Lipid umfasst.

13. Formulierung gemäß einem der Ansprüche 1-11, wobei die Formulierung einen chemischen Penetrationsverstärker umfasst, der die Wirksamkeit der Immunisierung im Vergleich zu der Verwendung einer Formulierung ohne den chemischen Penetrationsverstärker erhöht.

14. Formulierung gemäß einem der Ansprüche 1-13, wobei Exponieren der intakten Haut gegenüber dem Hilfsstoff eine darunter liegende Langerhans-Zelle aktiviert.

15. Formulierung gemäß einem der Ansprüche 1-14, wobei Exponieren der intakten Haut gegenüber dem Hilfsstoff bewirkt, dass eine darunter liegende Langerhans-Zelle die Expression des Major-Histocompatibility-Complex-Class II erhöht.

16. Formulierung gemäß einem der Ansprüche 1-14, wobei Exponieren der intakten Haut gegenüber dem Hilfsstoff das Wandern einer darunter liegenden Langerhans-Zelle zu einem Lymphknoten bewirkt.

17. Formulierung gemäß einem der Ansprüche 1-14, wobei Exponieren der intakten Haut gegenüber dem Hilfsstoff einer darunter liegenden Langerhans-Zelle das Signal zum Reifen zu einer Antigenpräsentierenden Zelle gibt.

18. Formulierung gemäß einem der Ansprüche 1-14, wobei Exponieren der intakten Haut gegenüber dem Hilfsstoff die Antigen-Präsentation an Lymphozyten erhöht.

19. Formulierung gemäß einem der vorstehenden Ansprüche, ferner umfassend einen Exzipienten, einen Stabilisator, ein Trockenmittel oder ein Konservierungsmittel.

20. Formulierung gemäß Anspruch 19, umfassend ein Konservierungsmittel oder einen Stabilisator, ausgewählt aus der Gruppe, bestehend aus Sucrose, Mannitol, Sorbitol, Trehalose, Dextran und Glycerin.

21. Formulierung gemäß einem der Ansprüche 1-20, ferner umfassend ein Verbandsmaterial, wodurch eine Pflasterform der Formulierung bereitgestellt wird.

22. Formulierung gemäß Anspruch 21, wobei das Verbandsmaterial ein okklusives Verbandsmaterial ist.

23. Formulierung gemäß Anspruch 21, wobei die intakte Haut, die von dem Verbandsmaterial bedeckt wird, eine Oberfläche aufweist, die größer als das Gebiet wenigstens eines drainierenden Lymphknotens ist.

24. Trockene Formulierung gemäß einem der vorstehenden Ansprüche zur transkutanen Immunisierung durch Auftragen der Formulierung auf die intakte Haut eines Subjekts ohne Perforation davon, umfassend das Verstärken der Penetration von wenigstens einem Antigen oder Hilfsstoff durch die Haut mit wenigstens einem physikalischen oder chemischen Penetrationsverstärker, der vor, nach und/oder während des Auftragens der Formulierung auf die Haut auf die Haut aufgetragen wird.

25. Trockene Formulierung gemäß Anspruch 24, umfassend das Auftragen von Alkohol auf die intakte Haut oder Hydratisieren davon vor dem Auftragen der Formulierung.

26. Verfahren zum Herstellen einer Formulierung gemäß einem der Ansprüche 1-23 zur transkutanen Immunisierung, umfassend:
(a) Bereitstellen von wenigstens einem Antigen und wenigstens einem Hilfsstoff;
(b) Auflösen von wenigstens dem Antigen und dem Hilfsstoff, um eine immunologisch aktive Flüssigkeit herzustellen;
(c) Trocknen der immunologisch aktiven Flüssigkeit auf ein festes Substrat, um die Formulierung herzustellen;
(d) wobei wenigstens das Antigen und der Hilfsstoff wenigstens bis zum Auftragen der Formulierung auf Haut eines zu immunisierenden Subjekts in trockener Form vorliegen, und wobei der Hilfsstoff ein bakterielles ADP-ribosylierendes Exotoxin oder eine Gangliosid-GM1-bindende Untereinheit davon umfasst.

27. Verfahren gemäß Anspruch 26, ferner umfassend das Zusammenmischen aller aktiven Inhaltsstoffe, um eine homogene Formulierung herzustellen.

28. Verfahren gemäß Anspruch 26 oder 27, wobei die Formulierung im Wesentlichen aus einem immunologisch aktiven Inhaltsstoff besteht.

29. Verfahren gemäß einem der Ansprüche 26-28, wobei das feste Substrat ein Verbandsmaterial ist und die Formulierung **dadurch** als Pflaster bereitgestellt wird.

30. Verfahren gemäß einem der Ansprüche 26-29, wobei die Formulierung unter aseptischen Bedingungen, die für die Herstellung eines Impfstoffs geeignet sind, hergestellt wird.

31. Verfahren gemäß einem der Ansprüche 26-30, wobei einzelnes Auftragen der Formulierung ausreicht, um eine nachweisbare Antigen-spezifische Immunantwort zu induzieren.

32. Verfahren gemäß einem der Ansprüche 26-31, wobei die Formulierung ferner wenigstens ein/einen Exzipienten, Stabilisierungsmittel, Trockenmittel oder Konservierungsmittel umfasst.

## Revendications

1. Formulation pour immunisation transcutanée, comprenant un antigène sous forme sèche et un adjuvant sous forme sèche, l'application de la formulation sur la peau intacte, sans perforation de cette dernière, conduisant à une réponse immune qui est spécifique de l'antigène, ledit adjuvant comprenant une exotoxine bactérienne de ribosylation de l'ADP ou une sous-unité de liaison au ganglioside GM1 de cette dernière.

2. Formulation selon la revendication 1, dans laquelle ledit antigène a une masse moléculaire supérieure à 500 Da.

3. Formulation selon la revendication 1, dans laquelle ledit antigène dérive d'un micro-organisme pathogène choisi dans le groupe consistant en les bactéries, les virus, les champignons et les parasites.

4. Formulation selon la revendication 3, dans laquelle l'antigène est choisi dans le groupe consistant en Bacillus anthracis, Campylobacter, Vibrio cholerae, Clostridia, comprenant Clostridium difficile, Diphteria, E. coli entérohémorragique, E. coli entérotoxigène, Giardia, Gonococcus, Helicobacter pylori ou l'uréase produite par H. pylori, Hemophilus influenza B, Hemophilus influenza non typable, Legionella, Meningococcus, Mycobacterium, comprenant les organismes responsables de la tuberculose, de la coqueluche, Pneumococcus, Salmonella, Shigella, Staphylococcus et leurs entérotoxines, Streptococcus bêta-hémolytique du groupe A, Streptococcus B, tétanos, Borrelia burgdorfi et Yersinia.

5. Formulation selon la revendication 3, dans laquelle l'antigène est choisi dans le groupe consistant en les adénovirus, les sérotypes 1 à 4 de la dengue, Ebola, les entérovirus, le virus Hanta, les sérotypes A à E de l'hépatite, le virus de l'herpès simplex 1 ou 2, le virus de l'immunodéficience humaine, le virus du papillome humain, la grippe, la rougeole, le virus de Norwalk, l'encéphalite équine du Japon, les papillomavirus, le parvovirus B19, le virus de la poliomyélite, la rage, le virus respiratoire syncytial, les rotavirus, la rougeole, la rubéole, l'encéphalite de Saint Louis, la vaccine, les vecteurs d'expression virale contenant des gènes codant pour d'autres antigènes tels que les antigènes de la malaria, la varicelle et la fièvre jaune.

6. Formulation selon la revendication 1, dans laquelle ledit antigène est un antigène tumoral ou un auto-antigène.

7. Formulation selon la revendication 1, dans laquelle ledit antigène est choisi dans le groupe consistant en les hydrates de carbone, les glycolipides, les glycoprotéines, les lipides, les lipoprotéines, les phospholipides et les polypeptides.

8. Formulation selon la revendication 1, dans laquelle la formulation est constituée d'un virus vivant atténué, et au moins un antigène est exprimé par le virus vivant atténué.

9. Formulation selon la revendication 1, dans laquelle ledit antigène est un polypeptide ayant une masse moléculaire supérieure à 500 Da.

10. Formulation selon la revendication 1, dans laquelle ledit antigène est multivalent.

11. Formulation selon la revendication 1, dans laquelle une molécule unique est simultanément un adjuvant et un antigène de la formulation.

12. Formulation selon l'une quelconque des revendications 1 à 7 ou 9 à 11, la formulation ne comprenant pas de renforçateur de pénétration, de particules virales, de liposomes ou des lipides chargés.

13. Formulation selon l'une quelconque des revendications 1 à 11, la formulation comprenant un renforçateur de pénétration chimique, qui augmente l'efficacité de l'immunisation par comparaison avec l'utilisation d'une formulation ne contenant pas le renforçateur de pénétration chimique.

14. Formulation selon l'une quelconque des revendications 1 à 13, pour laquelle l'exposition de la peau intacte à l'adjuvant active une cellule de Langerhans sous-jacente.

15. Formulation selon l'une quelconque des revendications 1 à 14, pour laquelle l'exposition de la peau intacte à l'adjuvant provoque une augmentation, par une cellule de Langerhans sous-jacente, de l'expression du complexe majeur d'histocompatibilité de classe II.

16. Formulation selon l'une quelconque des revendications 1 à 14, pour laquelle l'exposition de la peau intacte à l'adjuvant provoque la migration d'une cellule de Langerhans sous-jacente vers un ganglion lymphatique.

17. Formulation selon l'une quelconque des revendications 1 à 14, pour laquelle l'exposition de la peau intacte à l'adjuvant donne à une cellule de Langerhans sous-jacente une instruction de maturation en une cellule présentant l'antigène.

18. Formulation selon l'une quelconque des revendications 1 à 14, pour laquelle l'exposition de la peau intacte à l'adjuvant renforce la présentation de l'antigène à des lymphocytes.

19. Formulation selon l'une quelconque des revendications précédentes, qui comprend en outre un excipient, un stabilisant, un déshydratant ou un conservateur.

20. Formulation selon la revendication 19, qui comprend un conservateur ou un stabilisant choisi dans le groupe consistant en le saccharose, le mannitol, le sorbitol, le tréhalose, le dextran et le glycérol.

21. Formulation selon l'une quelconque des revendications 1 à 20, qui comprend en outre un pansement, de façon à réaliser une forme "timbre transdermique" de la formulation.

22. Formulation selon la revendication 21, dans laquelle le pansement est un pansement occlusif.

23. Formulation selon la revendication 21, pour laquelle la peau intacte recouverte par le pansement a une aire qui est plus grande que celle d'une zone de ganglion lymphatique de drainage.

24. Formulation sèche selon l'une quelconque des revendications précédentes, pour immunisation transcutanée par application de la formulation sur la peau intacte d'un sujet sans perforation de cette dernière, et comprenant le renforcement de la pénétration d'au moins un antigène ou d'un adjuvant à travers la peau, avec au moins un renforçateur de pénétration physique ou chimique appliqué sur la peau avant, après et/ou pendant l'application de la formulation sur la peau.

25. Formulation sèche de la revendication 24, comprenant l'application d'alcool sur la peau intacte, ou la déshydratation de cette dernière, avant application de la formulation.

26. Procédé de production d'une formulation selon l'une quelconque des revendications 1 à 23 pour immunisation transcutanée, comprenant :
(a) la mise à disposition d'au moins un antigène et d'au moins un adjuvant ;
(b) la dissolution d'au moins l'antigène et l'adjuvant, pour produire un liquide immunologiquement actif ;
(c) le séchage du liquide immunologiquement actif sur un substrat solide pour produire la formulation ;
(d) où au moins l'antigène et l'adjuvant se présentent sous forme sèche au moins jusqu'à application de la formulation sur la peau d'un sujet à immuniser, l'adjuvant comprenant une exotoxine bactérienne de ribosylation de l'ADP ou une sous-unité de liaison du ganglioside GM1 de cette dernière.

27. Procédé selon la revendication 26, qui comprend en outre le mélange de tous les principes actifs les uns avec les autres pour produire une formulation homogène.

28. Procédé selon la revendication 26 ou 27, dans lequel la formulation est constituée essentiellement d'un ingrédient immunologiquement actif.

29. Procédé selon l'une quelconque des revendications 26 à 28, dans lequel le substrat solide est un pansement, et la formulation est de ce fait mise à disposition sous forme d'un timbre transdermique.

30. Procédé selon l'une quelconque des revendications 26 à 29, dans lequel la formulation est produite dans des conditions aseptiques convenant à la production d'un vaccin.

31. Procédé selon l'une quelconque des revendications 26 à 30, dans lequel une application unique de la formulation est suffisante pour induire une réponse immune spécifique d'antigène détectable.

32. Procédé selon l'une quelconque des revendications 26 à 31, dans lequel la formulation est en outre constituée d'au moins un excipient, un stabilisant, un déshydratant ou un conservateur.
